# EUROPEAN PATENT APPLICATION

(11) **EP 3 202 902 A1**
(43) Date of publication of application: **09.08.2017**
(21) Application number: 15847174.8
(22) Date of filing: 28.09.2015
(51) Int. Cl.: C12N 15/09, C12Q 1/68, G01N 33/53, G01N 33/543, C07K 16/00

(54) **METHOD AND KIT FOR MEASURING TARGET NUCLEIC ACID CONTAINING MODIFIED NUCLEOBASE**

(30) Priority: 29.09.2014 JP 2014197719
(71) Applicant: Fujirebio Inc., Shinjuku-ku Tokyo 163-0410 (JP)
(72) Inventor: MATSUNO, Tatsuki, Tokyo 163-0410 (JP); HORIIKE, Mariko, Tokyo 163-0410 (JP)
(74) Representative: Hoffmann Eitle
(86) International application number: PCT/JP2015/077233
(87) International publication number: WO 2016/052368

(57) **Abstract**

The present invention provides a method and means that can specifically detect a target nucleic acid containing a modified nucleobase.

Specifically, the present invention provides a method for measuring a target nucleic acid containing a modified nucleobase, the method comprising:
(1) incubating a nucleic acid sample and a heterologous nucleic acid probe having a property of pairing with a modified nucleobase, in a solution; and
(2) measuring the modified nucleobase using an antibody against a modified nucleobase having a property of heterologous pairing, in the solution obtained at (1).

The present invention also provides a kit for measuring a target nucleic acid containing a modified nucleobase, the kit comprising:
(I) a heterologous nucleic acid probe having a property of pairing with a modified nucleobase; and
(II) an antibody against a modified nucleobase having a property of heterologous pairing.

## Description

### TECHNICAL FIELD

The present invention relates to a method for measuring a target nucleic acid containing a modified nucleobase and a kit.

### BACKGROUND ART

There are some reports about a technique that detects a naturally occurring modified nucleobase (e.g., methylcytosine and hydroxy methylcytosine) by immunoassays (see, for example, Patent Literature 1 and Non Patent Literatures 1 and 2).

### PRIOR ART LITERATURE

### Patent Literature

Patent Literature 1: JP-2012-230019-A

Non Patent Literature 1: Proll et al., DNA Research, 13, 37-42 (2006)
Non Patent Literature 2: Kurita et al., Anal. Chem., 2012, 84, 7533-7538

### DISCLOSURE OF INVENTION

### PROBLEM TO BE SOLVED BY THE INVENTION

In conventional methods, antibodies which cannot recognize modified nucleobases that is pairing with nucleobases but can recognize modified nucleobases in the unpaired state has been used. Accordingly, in order to measure modified nucleobases existing in particular positions of target nucleic acids in the conventional methods using such antibodies, it was necessary to allow the modified nucleobases to be unpaired. Examples of such methods include: 1) a method comprising a) fragmenting a target nucleic acid so that only a modified nucleobase to be measured is included, and then b) measuring the modified nucleobase in the target nucleic acid by using the fragmented target nucleic acid (single-stranded nucleic acid) and an antibody against the modified nucleobase (a method for measuring a modified nucleobase in a single-stranded nucleic acid); and 2) a method comprising a) forming a double-stranded nucleic acid of a target nucleic acid containing a modified nucleobase with a nucleic acid probe by using a given nucleic acid probe (in the double-stranded nucleic acid, the modified nucleobase is unpaired), and then b) measuring the modified nucleobase in the target nucleic acid by using the double-stranded nucleic acid and an antibody against the modified nucleobase (a method for measuring a modified nucleobase in a double-stranded nucleic acid).

As the method of 1), there is a method in which a cleavage with a restriction enzyme is utilized. However, a restriction enzyme that allows a modified nucleobase to be measured to exclusively be included in a cleaved fragment is not always available.

Examples of the step a) in the method 2) include: i) a method for masking a modified nucleobase not to be measured by binding a nucleic acid probe capable of pairing with a modified nucleobase not to be measured existing in a target nucleic acid, to the target nucleic acid; and ii) a method for allowing a modified nucleobase to be measured to be in the unpaired state by binding a nucleic acid probe incapable of pairing with a modified nucleobase to be measured existing in a target nucleic acid, to the target nucleic acid (formation of a bulge structure). However, method i) has a problem that an extremely long nucleic acid probe is required for masking all of the modified nucleobases not to be measured, and the synthesis thereof is difficult. A method of binding a plurality of short chain nucleic acid probes can also be considered. However, there is a problem that these nucleic acid probes may form a complex to each other, and thus it is difficult to bind the probes specifically and efficiently to the target nucleic acid. Moreover, there is also a problem that it is difficult to completely mask all of the modified nucleobases not to be measured. In addition, method ii) has a problem that when a bulge structure is formed at the part of a modified nucleobase to be measured, a mismatch between the nucleic acid probe and the target nucleic acid occurs, resulting in a decrease of specificity of the nucleic acid probe, which in turn leads to a decrease of specificity of the measurement.

Specifically, in Patent Literature 1, and in Non Patent Literatures 1 and 2, methylcytosine is detected by using the anti-methylcytosine antibody (clone 33D3). Although the anti-methylcytosine antibody can recognize unpaired methylcytosine, it cannot recognize methylcytosine pairing with the complementary nucleobase (guanine). Accordingly, in the methods of Patent Literature 1, and Non Patent Literatures 1 and 2, in which such antibodies are used, a modified nucleobase existing in a particular position of a target nucleic acid must be unpaired in order to measure the modified nucleobase, which results in the problems described above.

The immunoassay system described above has a problem that not only a target nucleic acid containing a modified nucleobase, but also a non-target nucleic acid containing the modified nucleobase may be detected. This is caused by that since the antibodies against modified nucleobases used in the immunoassays described above recognize only unpaired modified nucleobases, modified nucleobases will be recognized by those antibodies with no distinction whether the modified nucleobases are contained in target nucleic acids or in non-target nucleic acids. In particular, this problem could be serious when a naturally occurring sample to be measured which may include a modified nucleobase that is contained not only in a target nucleic acid, but also in many non-target nucleic acids. An object of the present invention is to specifically measure a target nucleic acid containing a modified nucleobase, particularly without requiring processes such as fragmentation of the target nucleic acid and masking of modified nucleobases not to be measured (5-methylcytosine).

### MEANS FOR SOLVING PROBLEM

As a result of intensive investigations, the inventors of the present invention conceived of the idea that in order to specifically measure a target nucleic acid containing a modified nucleobase, specificity of measurement can be improved by using an antibody that can recognize the modified nucleobase pairing with the nucleobase but cannot recognize the unpaired modified nucleobases, differently from conventional antibodies. That is, by using such an antibody, and by properly designing a nucleic acid probe, a modified nucleobase in a target nucleic acid that is hybridized with the nucleic acid probe can specifically be measured. The inventors of the present invention also conceived of using a nucleic acid probe that can pair with a modified nucleobase for adaptation to the use of such an antibody; and utilizing a heterologous nucleic acid as a nucleic acid included in the nucleic acid probe for improved accuracy of measurement. In fact, the inventors of the present invention have succeeded to specifically measure a target nucleic acid containing a modified nucleobase by using a method developed based on such conception, and have achieved the present invention.

That is, the present invention is as follows.
[1] A method for measuring a target nucleic acid containing a modified nucleobase, the method comprising:
   (1) incubating a nucleic acid sample and a heterologous nucleic acid probe having a property of pairing with a modified nucleobase, in a solution; and
   (2) measuring the modified nucleobase using an antibody against a modified nucleobase having a property of heterologous pairing, in the solution obtained at (1).
[2] The method according to [1], wherein
   the nucleic acid sample contains the target nucleic acid containing the modified nucleobase, and
   steps (1) and (2) are performed by (1') and (2'), respectively:
   (1') reacting the nucleic acid sample containing the target nucleic acid containing the modified nucleobase with the heterologous nucleic acid probe having a property of pairing with the modified nucleobase, in a solution by incubation, to form a heterologous nucleic acid hybrid including said target nucleic acid and said probe; and
   (2') measuring the modified nucleobase using said antibody in the solution containing said heterologous nucleic acid hybrid.
[3] The method according to [1] or [2], further comprising adding said probe to a solution containing said nucleic acid sample to prepare a solution containing both of said nucleic acid sample and said probe.
[4] The method according to any of [1] to [3], wherein said nucleic acid sample is a sample containing a target DNA containing a modified nucleobase.
[5] The method according to any of [1] to [4], wherein said probe contains an artificial nucleic acid having a main chain structure different from a main chain structure of the target nucleic acid.
[6] The method according to [5], wherein said probe is a peptide nucleic acid (PNA) probe or a bridged nucleic acid (BNA) probe.
[7] The method according to any of [1] to [6], wherein a nucleobase included in the modified nucleobase is cytosine.
[8] The method according to any of [1] to [7], wherein the modified nucleobase is methylcytosine.
[9] A method for measuring a target nucleic acid containing a modified nucleobase, the method comprising:
   (1) incubating a nucleic acid sample, a heterologous guide probe having a property of pairing with a modified nucleobase, and a capture probe in a solution; and
   (2) measuring the modified nucleobase using an antibody against a modified nucleobase having a property of heterologous pairing, in the solution obtained at (1).
[10] The method according to [9], wherein
   the nucleic acid sample contains the target nucleic acid containing the modified nucleobase, and
   steps (1) and (2) are performed by (1') and (2'), respectively:
   (1') reacting the nucleic acid sample, the heterologous guide probe having a property of pairing with a modified nucleobase, and the capture probe in a solution by incubation to form a heterologous nucleic acid hybrid including said target nucleic acid, said guide probe, and the capture probe; and
   (2') measuring the modified nucleobase using the antibody in the solution containing said heterologous nucleic acid hybrid.
[11] The method according to [10], wherein said guide probe is heterologous to said target nucleic acid; and the capture probe is heterologous to both of said target nucleic acid and said guide probe.
[12] The method according to any of [1] to [11], wherein the measurement of the target nucleic acid containing the modified nucleobase using said antibody is performed by ELISA.
[13] A kit for measuring a target nucleic acid containing a modified nucleobase, the kit comprising:
   (I) a heterologous nucleic acid probe having a property of pairing with a modified nucleobase; and
   (II) an antibody against a modified nucleobase having a property of heterologous pairing.
[14] The kit for measuring according to [13], wherein said probe is a heterologous guide probe having a property of pairing with the modified nucleobase, and the kit further comprises (III) a capture probe.

### EFFECT OF THE INVENTION

With the method and the kit according to the present invention, a modified nucleobase existing in a target nucleic acid can specifically be measured. Specifically, according to the present invention in which a heterologous nucleic acid probe having a property of pairing with a modified nucleobase and an antibody against a modified nucleobase having a property of heterologous pairing are used, a modified nucleobase existing on a portion where a hybrid is formed with the probe can specifically be measured. According to the present invention, processes such as fragmentation of the target nucleic acid and masking of modified nucleobases not to be measured are not required.

### BRIEF DESCRIPTION OF DRAWINGS

FIG. 1 illustrates a specific detection of a modified nucleobase using an antibody against a modified nucleobase having a property of heterologous pairing and a nucleic acid probe (e.g., a heterologous nucleic acid probe having a property of pairing with a modified nucleobase).
FIG. 2 illustrates a peripheral sequence independent detection of a modified nucleobase using an antibody against a modified nucleobase having a property of heterologous pairing and a heterologous nucleic acid probe having a property of pairing with a modified nucleobase.
FIG. 3 illustrates a dose dependent detection of a modified nucleobase using an antibody against a modified nucleobase having a property of heterologous pairing and a heterologous nucleic acid probe having a property of pairing with a modified nucleobase.
FIG. 4 illustrates a specific detection of a modified nucleobase using an antibody against a modified nucleobase having a property of heterologous pairing, a heterologous guide probe pairing with a modified nucleobase, and a capture probe.
FIG. 5 illustrates a specific detection of a modified nucleobase using an antibody against a modified nucleobase having a property of heterologous pairing and a nucleic acid probe having a property of pairing with a modified nucleobase (BNA probe) (1).
FIG. 6 illustrates a specific detection of a modified nucleobase using an antibody against a modified nucleobase having a property of heterologous pairing and a nucleic acid probe having a property of pairing with a modified nucleobase (BNA probe) (2).
FIG. 7 illustrates an overview of a method according to the present invention.

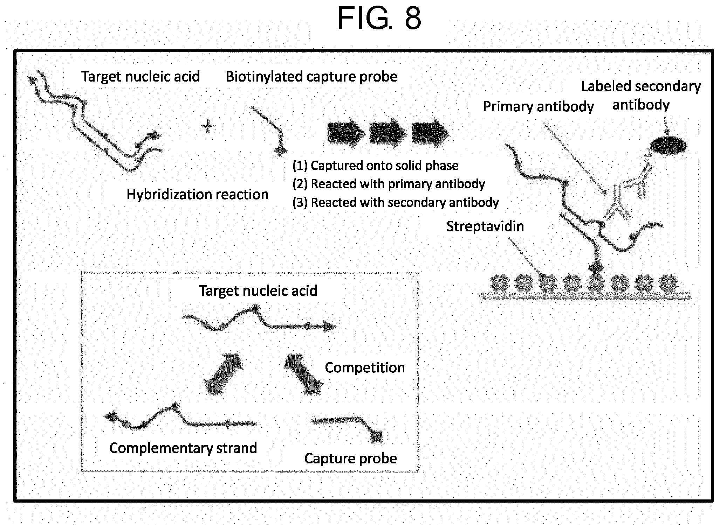

FIG. 8 illustrates an overview of a problem (Specific Problem I) related to the measurement of a modified nucleobase. Detection sensitivity for a modified nucleobase in a double-stranded target nucleic acid is lower than that for the modified nucleobase in a single-stranded target nucleic acid. This is because it is considered that a complementary chain and a capture probe compete against each other for the target nucleic acid containing the modified nucleobase and that hybrid formation efficiency between the target nucleic acid and the capture probe (efficiency of capturing the target nucleic acid to a solid phase) is low.

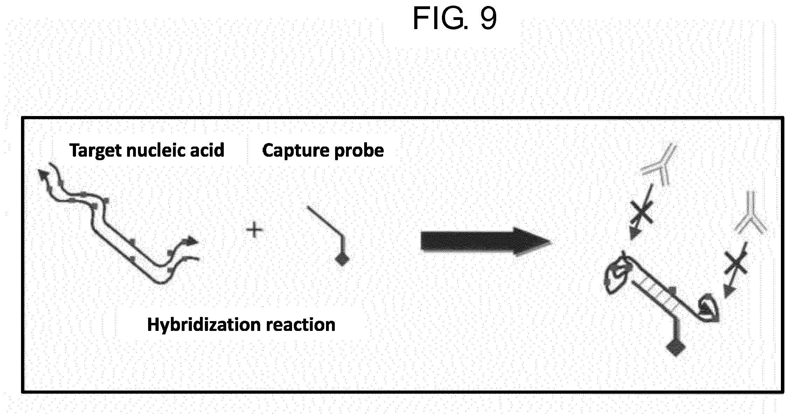

FIG. 9 illustrates an overview of a problem (Specific Problem II) related to the measurement of the modified nucleobase. In a conventional method for measuring the modified nucleobase in the target nucleic acid using the capture probe, a hybrid including the target nucleic acid and the capture probe is formed. The conventional method has a potential problem in that a non-hybridized region (single-stranded region) in this hybrid forms a secondary structure, whereby the modified nucleobase contained in this secondary structure is difficult to be measured (in other words, detection sensitivity is low).
FIG. 10 illustrates the measurement of the modified nucleobase in the single-stranded and double-stranded target nucleic acids by the capture probe.
FIG. 11 illustrates the measurement of the modified nucleobase in the single-stranded target nucleic acid using the capture probe and a guide probe. Guide probe (-): the capture probe alone; and guide probe (+): the capture probe and the guide probe.
FIG. 12 illustrates the measurement of the modified nucleobase in the double-stranded target nucleic acid using the capture probe and the guide probe. Guide probe (-): the capture probe alone; and guide probe (+): the capture probe and the guide probe.
FIG. 13 illustrates the measurement of the modified nucleobase in the double-stranded target nucleic acid using the guide probe in the presence of a chaotropic agent. Hybridization buffer: the guide probe is used; guanidine thiocyanate (-) buffer: the guide probe is used; and guanidine thiocyanate (+) buffer: the guide probe is used in the presence of the chaotropic agent.
FIG. 14 illustrates the measurement of the modified nucleobase in the single-stranded and double-stranded target nucleic acids using the guide probe in the presence of the chaotropic agent.
FIG. 15 illustrates the measurement of the modified nucleobase in the single-stranded and double-stranded target nucleic acids using the guide probe. This experiment was carried out for comparison with the experiment illustrated in FIG. 14.
FIG. 16 illustrates the measurement of the modified nucleobase using the guide probe in the presence of a nucleic acid denaturant.
FIG. 17 illustrates the inhibition of the formation of a secondary structure in a site containing the modified nucleobase by the guide probe. 1: Guide Probe 1; 2: Guide Probe 2; 3: Guide Probe 3; 4: Guide Probe 4; 2+4; Guide Probes 2 and 4 are added; and 2+3+4; Guide Probes 2, 3, and 4 are added.
FIG. 18 illustrates the effect of the nucleic acid denaturant at various concentrations.
FIG. 19 illustrates the investigation of the main chain of the guide probe. No addition: in the absence of the guide probe; 2: Guide Probe 2; 4: Guide Probe 4; 2+4; in the presence of Guide Probes 2 and 4; 5: Guide Probe 5; 6: Guide Probe 6; 5+6: in the presence of Guide Probes 5 and 6; -: in the absence of the guide probe; DNA: the main chain of the guide probe is DNA; 2'-O-methylated RNA: the main chain of the guide probe is 2'-O-methylated RNA; and RNA: the main chain of the guide probe is RNA.
FIG. 20 illustrates the measurement of the modified nucleobase using the guide probe in the presence of the nucleic acid denaturant (the chaotropic agent and an electron donating compound) or a non-nucleic acid denaturant (surfactant).
FIG. 21 illustrates the measurement (luminescence counts) of the modified nucleobase in the target nucleic acid using the guide probe in the presence of both the nucleic acid denaturant and the surfactant.
FIG. 22 illustrates the measurement (S/N) of the modified nucleobase in the target nucleic acid using the guide probe in the presence of both the nucleic acid denaturant and the surfactant. Signal-to-noise ratio (S/N) : luminescence counts of an amount of the target nucleic acid (fmol)/luminescence counts in the absence (that is, 0 mol) of the target nucleic acid.

### EMBODIMENT FOR CARRYING OUT THE INVENTION

The present invention provides a method for measuring a target nucleic acid containing a modified nucleobase. The present invention includes:
(1) incubating a nucleic acid sample and a heterologous nucleic acid probe having a property of pairing with a modified nucleobase, in a solution; and
(2) measuring a modified nucleobase using an antibody against a modified nucleobase having a property of heterologous pairing, in the solution obtained at (1).

The nucleic acid sample is a sample containing a target nucleic acid containing a modified nucleobase or a sample suspected to contain the target nucleic acid. The nucleic acid sample may also be a biological sample derived from an organism, an environmental sample, or the like. Examples of the organism from which the biological sample is derived include animals such as mammals (e.g., humans, monkeys, mice, rats, rabbits, cattle, pigs, horses, goats, and sheep) and birds (e.g., chickens), insects, microorganisms, plants, fungi, and fishes. The biological sample may also be a blood-related sample that is blood itself or a blood-derived sample (e.g., whole blood, blood serum, or blood plasma), saliva, urine, milk, tissue or cell extract, or a mixture thereof. The biological sample may further be derived from mammals contracting diseases (e.g., cancer and leukemia) or mammals that may contract diseases. Examples of the environmental sample include samples derived from soil, sea water, and fresh water that may contain nucleic acids. These samples may be subjected to another treatment before being used in the method according to the present invention. Examples of the treatment include extraction and fragmentation (e.g., treatment with an enzyme such as a restriction enzyme) of nucleic acids (e.g., DNA such as genomic DNA and RNA). Accordingly, the method according to the present invention may further include extracting a nucleic acid from the nucleic acid sample, and/or fragmenting the nucleic acid. The method according to the present invention may also further include treating the sample by centrifugation, extraction, filtration, precipitation, heating, freezing, refrigeration, stirring, or the like.

The target nucleic acid is DNA or RNA, and preferably DNA. The target nucleic acid is also a coding region or a non-coding region (e.g., a transcriptional regulation region) of DNA. A portion or whole of a nucleotide sequence of the target nucleic acid is complementary to a nucleotide sequence of the heterologous nucleic acid probe. The number of nucleotide residues included in the target nucleic acid (that is, the length of the target nucleic acid) is not limited to a particular number so long as it enables hybridization with the heterologous nucleic acid probe and may be 10 or more, preferably 15 or more, or more preferably 20 or more, for example. The number of nucleotides included in the target nucleic acid is not particularly limited, and may also be any number that may occur by fragmentation of genomic DNA, for example. The number of the nucleotides included in the target nucleic acid may be 10,000 or less, 5,000 or less, 2,000 or less, 1,000 or less, 500 or less, 200 or less, or 100 or less, for example. A GC content of the target nucleic acid is not limited to a particular value and may be 10% or more, 20% or more, 30% or more, 40% or more, 50 % or more, or 60% or more, for example. The GC content of the target nucleic acid may also be 90% or less, 80% or less, or 70% or less, for example. The number of modified nucleobases that the target nucleic acid contains is not limited to a particular number so long as it is one or more (e.g., 1 to 100, 1 to 20, 1 to 10, or 1 to 5).

In the present invention, the modified nucleic acid refers to a nucleobase having a structure in which a normal nucleobase selected from the group consisting of adenine (A), guanine (G), cytosine (C), thymine (T), and uracil (U) is modified. When the target nucleic acid is DNA, examples of the term "nucleobase" in the expression "modified nucleobase" include adenine (A), guanine (G), cytosine (C), and thymine (T). When the target nucleic acid is RNA, examples thereof include adenine (A), guanine (G), cytosine (C), and uracil (U). The nucleobase is preferably cytosine (C). Examples of modification include introduction of a substituent to the normal nucleobase, elimination of a group that the normal nucleobase has (e.g., an amino group, an oxo group, and a methyl group), and exchange of the group that the normal nucleobase has with a substituent. The substituent is not limited to a particular type so long as it is one that naturally occurring nucleobases can have, and examples thereof include the substituents that the modified nucleobases in the modified nucleotides described in Administrative Instructions under the Patent Cooperation Treaty (the version enforced on January 1, 2009), Annex C, Appendix 2, Table 2: List of Modified Nucleotides have. The modified nucleotides described in the literature can be the same as the modified nucleotides described in "Guidelines for Preparation of Specifications Containing Base Sequences or Amino Acid Sequences (July of 2002) or (December of 2009)," Annex 2, Table 2: Modified Base Table disclosed by the Japan Patent Office. Accordingly, concerning the modified nucleobase, the above guidelines can also be referred to. The substituent is preferably methyl or hydroxymethyl. The position of the modification such as substitution is not limited to a particular position, and is the 2-position or the 4- to 6-positions, for example, and preferably the 5-position for the nucleobase having a pyrimidine ring (C, T, or U); and is the 2-position, the 6-position, or the 8-position, for example, for the nucleobase having a purine ring (A or G).

The modified nucleobase is not limited to a particular type so long as it can naturally occur, and examples thereof include the modified nucleobases that the modified nucleotides described in Administrative Instructions under the Patent Cooperation Treaty (the version enforced on January 1, 2009), Annex C, Appendix 2, Table 2: List of Modified Nucleotides have. The modified nucleotides described in the literature can be the same as the modified nucleotides described in the above guidelines, Annex 2, Table 2: Modified Base Table. Accordingly, concerning the modified nucleobase, the above guidelines can also be referred to. The modified nucleobase is preferably methylcytosine (e.g., 5-methylcytosine), hydroxymethylcytosine (e.g., 5-hydroxymethylcytosine), or carboxycytosine (e.g., 5-carboxycytosine). The modified nucleobase is more preferably methylcytosine (e.g., 5-methylcytosine) or hydroxymethylcytosine (e.g., 5-hydroxymethylcytosine). It is known that the modified nucleobase brings about changes in functions of nucleic acids (e.g., a change in the transcriptional regulation capability of a certain gene).

### (Heterologous nucleic acid probe having a property of pairing with a modified nucleobase: Probe X)

The heterologous nucleic acid probe having a property of pairing with a modified nucleobase used in the present invention refers to a heterologous nucleic acid probe which includes a nucleic acid heterologous to the target nucleic acid, which can detect the target nucleic acid by hybridization, and which contains nucleobases capable of pairing with a modified nucleobase when it forms a heterologous nucleic acid hybrid with the target nucleic acid. Hereinafter, the heterologous nucleic acid probe having a property of pairing with a modified nucleobase may simply be referred to as the probe X. Accordingly, a modified nucleobase pairs with a complementary nucleobase in the heterologous nucleic acid hybrid formed from the target nucleic acid and the probe X, and thus neither a bulge structure nor a loop structure is formed at the part of the modified nucleobase. Specifically, when the modified nucleobase is adenine (A), a pairing nucleobase in the probe X is thymine (T) or uracil (U). When the modified nucleobase is guanine (G), a pairing nucleobase in the probe X is cytosine (C). When the modified nucleobase is cytosine (C), a pairing nucleobase in the probe X is guanine (G). When the modified nucleobase is thymine (T) or uracil (U), a pairing nucleobase in the probe X is adenine (A). The term "heterologous" means that the probe X has a main chain structure different from the main chain structure (the structure including the sugar moiety and the phosphoric acid moiety) of the target nucleic acid as a portion or whole of the main chain structure. Accordingly, the type of the probe X is determined in accordance with the type of the target nucleic acid. When the target nucleic acid is DNA, for example, a nucleic acid probe other than a DNA probe can be used as the probe X. When the target nucleic acid is natural RNA, a nucleic acid probe other than a normal RNA probe including RNA homologous with the natural RNA can be used as the probe X. Since DNA is preferred as the target nucleic acid, the probe X is preferably a nucleic acid probe other than a DNA probe.

Examples of the probe X include DNA probes, RNA probes, peptide nucleic acid (PNA) probes, locked nucleic acid (LNA) probes or bridged nucleic acid (BNA) probes, phosphorothioate (S-oligo) nucleic acid probes, and chimera nucleic acid probes in which two or more such nucleic acid probes are coupled with each other (the chimera nucleic acid probe inevitably include a nucleic acid heterologous to the target nucleic acid).

A preferable example of the structural unit of the bridged nucleic acid (BNA) is represented by the formula of below.

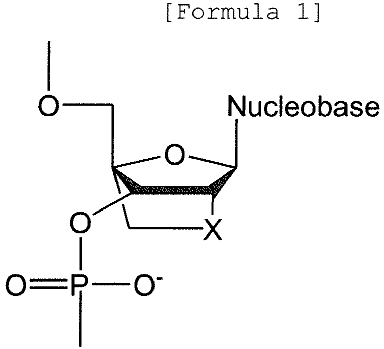

In the general formula above, X represents a linker (an atom or a group). Examples of the linker (an atom or a group) include -O-, -O-CH₂-O-, and -NR-O-. Examples of the R include a hydrogen atom and a hydrocarbon group [e.g., an alkyl group such as a C₁₋₆ alkyl group, as will be mentioned later]. The "Nucleobase" represents a nucleobase described above. The BNA in which X is -N(CH₃)-O- may be described as BNA-NC (N-Me).

Examples of the RNA probes include a normal RNA probe including a natural ribonucleotide having a hydroxy group at the 2'-position and a modified RNA probe including a ribonucleotide the 2'-position hydroxy group of which is modified. As the modified RNA probe, a ribonuclease-resistant RNA probe may be used. Examples of the modified RNA probe include a 2'-O-alkylated RNA probe. The 2'-O-alkylated RNA probe is preferably 2'-O-C₁₋₆ alkylated RNA probe. The C₁₋₆ alkyl group of the C₁₋₆ alkylation is a linear, branched, or cyclic C₁₋₆ alkyl group, and examples thereof include a methyl group, an ethyl group, a propyl group (e.g., n-propyl and iso-propyl), a butyl group (e.g., n-butyl, iso-butyl, sec-butyl, and tert-butyl), a pentyl group, and a hexyl group. In terms of easiness of manufacture and obtainment or the like, the 2'-O-C₁₋₆ alkylated RNA probe is a 2'-O-methylated RNA probe.

Preferably, the probe X may contain an artificial nucleic acid having a partial structure different from that of a natural nucleic acid. Such a probe X may preferably contain an artificial nucleic acid having a main chain structure different from the main chain structure (structure including a sugar moiety and a phosphoric acid moiety) of the target nucleic acid. Examples of the artificial nucleic acid having such a main chain structure include a structure in which a sugar moiety, which is the component moiety of the main chain structure, has been modified or replaced with another moiety; a structure in which a phosphoric acid moiety, which is the component moiety of the main chain structure, has been replaced with another moiety; and a structure in which both of the sugar moiety and the phosphoric acid moiety have been replaced with other moieties. When the probe X contains an artificial nucleic acid, all nucleic acids included in the probe X may be artificial nucleic acids. Alternatively, a nucleotide containing a nucleobase capable of pairing with a modified nucleobase when a heterologous nucleic acid hybrid is formed with the target nucleic acid and/or an adjacent nucleotide thereof (e.g., from the nucleotide containing a nucleobase capable of pairing with a modified nucleobase to two or one nucleotide(s) toward the 5'-end and/or the 3'-end) may be the artificial nucleic acid described above; however, the case in which only a nucleotide containing a nucleobase pairing with a modified nucleobase when a heterologous nucleic acid hybrid is formed with the target nucleic acid is the artificial nucleic acid described above is also preferable. Such a probe X further contains any nucleobases, and may preferably contain nucleobases selected from the group consisting of adenine (A), guanine (G), cytosine (C), thymine (T), and uracil (U).

More preferably, the probe X is i) a peptide nucleic acid (PNA) probe (A whole or portion of the probe is PNA. When a portion thereof is PNA, only a nucleotide containing a nucleobase capable of pairing with a modified nucleobase when a heterologous nucleic acid hybrid is formed with the target nucleic acid may be PNA.), ii) a bridged nucleic acid (BNA) probe (In this probe, a whole or portion thereof is BNA. When a portion thereof is BNA, only a nucleotide containing a nucleobase capable of pairing with a modified nucleobase when a heterologous nucleic acid hybrid is formed with the target nucleic acid may be BNA.), or iii) a chimera nucleic acid probe in which a PNA probe and/or BNA probe and another nucleic acid probe described above are linked.

The number of nucleotide residues included in the probe X (that is, the length of the probe X) is not limited to a particular number so long as it is long enough to be able to hybridize with the target nucleic acid and may be 10 or more, preferably 15 or more, and more preferably 20 or more, for example. The number of nucleotides included in the probe X may also be 100 or less, 80 or less, 60 or less, 50 or less, 40 or less, or 30 or less, for example. A GC content in the target nucleic acid is not particularly limited, and may be 10% or more, 20% or more, 30% or more, 40% or more, 50% or more, or 60% or more, for example. The GC content in the target nucleic acid may also be 90% or less, 80% or less, or 70% or less, for example. Such a probe X can be prepared by a method of synthesizing a probe known in the relevant field, for example.

The probe X is used in the form of being free or the form of being immobilized to the solid phase (this will be mentioned later). Accordingly, the probe X may be labeled with a substance or a group that enables immobilization to the solid phase. For example, the labeling is performed either at the 5'-end or the 3'-end. Examples of the substance or group that enables immobilization to the solid phase include groups or substances that enable covalent binding to the solid phase and affinity substances. Examples of the substances or groups that enable covalent binding to the solid phase include a thiol group or substances having a thiol group (the thiol group introduced into the probe X can bind to a maleimide group on the solid phase) and an amino group or substances having an amino group (the amino group introduced into the probe X can bind to maleic anhydride on the solid phase). Examples of the affinity substances include streptavidin, biotin, digoxigenin, dinitrophenol, fluorescein, and fluorescein isothiocyanate. In this case, the solid phase coated with another affinity substance having affinity with the affinity substance that the capture probe has can be used.

When the probe X is used in the form of being free, the probe X may be a heterologous guide probe having a property of pairing with a modified nucleobase. As probes in this case, a capture probe (probe for immobilizing the target nucleic acid to the solid phase) is used in addition to a heterologous guide probe having a property of pairing with a modified nucleobase (probe for helping detection of a modified nucleobase). Use of a combination of the heterologous guide probe having a property of pairing with a modified nucleobase and the capture probe is an embodiment of conferring the role of detecting a modified nucleobase and the role of immobilizing the target nucleic acid to the solid phase on other probes.

### (Heterologous guide probe: Probe Y)

The heterologous guide probe having a property of pairing with a modified nucleobase used in the present invention is a nucleic acid probe having the characteristics described above for the probe X, except that it is used exclusively in the form of being free. Hereinafter, the heterologous guide probe having a property of pairing with a modified nucleobase may simply be referred to as the probe Y. The probe Y can hybridize with the target nucleic acid in a region different from a region in the target nucleic acid with which the capture probe hybridizes. The probe Y is designed so as not to hybridize with the capture probe. One or a plurality of (e.g., two, three, four, or five) guide probes can be used against one target nucleic acid. The probes Y can be designed so as to hybridize with different regions within the one target nucleic acid, for example.

### (Capture probe)

The capture probe used in the present invention is a nucleic acid molecule having the capability of hybridizing with the target nucleic acid and can be immobilized to a solid phase. In the present invention, the capture probe is designed so as not to hybridize with the probe Y.

The capture probe can include nucleic acids homologous and/or heterologous with respect to the target nucleic acid. The term "homologous" means that the capture probe has the same main chain structure as a main chain structure (structure including a sugar moiety and a phosphoric acid moiety) of the target nucleic acid as the whole of the main chain structure. The term "heterologous" means that the capture probe has a main chain structure different from the main chain structure (the structure including the sugar moiety and the phosphoric acid moiety) of the target nucleic acid as a part or the whole of the main chain structure. Accordingly, the type of the capture probe may be determined in accordance with the type of the target nucleic acid. When the target nucleic acid is DNA, for example, a DNA probe can be used as the capture probe of the homologous nucleic acid, and a nucleic acid probe other than the DNA probe can be used as the capture probe of the heterologous nucleic acid. When the target nucleic acid is natural RNA, a normal RNA probe including RNA homologous with the natural RNA can be used as the capture probe of the homologous nucleic acid, and a nucleic acid probe other than the normal RNA probe can be used as the capture probe of the heterologous nucleic acid. The capture probe may preferably include the nucleic acid heterologous with respect to the target nucleic acid.

Examples of the capture probe include DNA probes, RNA probes, peptide nucleic acid (PNA) probes, locked nucleic acid (LNA) probes or bridged nucleic acid (BNA) probes, phosphorothioate (S-oligo) nucleic acid probes, and chimera nucleic acid probes in which two or more such nucleic acid probes are coupled with each other (the chimera nucleic acid probe inevitably contains a nucleic acid heterologous with respect to the target nucleic acid). RNA probes are the same as those described above.

The number of nucleotide residues included in the capture probe (that is, the length of the capture probe) is not limited to a particular number so long as it is long enough to be able to hybridize with the target nucleic acid and may be 12 or more, preferably 15 or more, preferably 18 or more, and more preferably 20 or more, for example. The number of nucleotides included in the capture probe may also be 100 or less, 80 or less, 60 or less, or 50 or less, for example. A GC content in a region that can hybridize with the target nucleic acid in the capture probe may be 10% or more, 20% or more, 30% or more, 40% or more, 50% or more, or 60% or more, for example. The GC content in this region may also be 90% or less, 80% or less, or 70% or less, for example. The capture probe can be prepared by a method of synthesizing a probe known in the relevant field, for example.

When the capture probe is used in Step (1), it is used in the form of being free or the form of being immobilized to the solid phase. Accordingly, the capture probe may be labeled with a substance or a group that enables immobilization to the solid phase. The labeling can be performed either at the 5'-end or the 3'-end of the capture probe, for example. Examples of the substance or group that enables immobilization to the solid phase include groups or substances that enable covalent binding to the solid phase and affinity substances. Examples of the substances or groups that enable covalent binding to the solid phase include a thiol group or substances having a thiol group (the thiol group introduced into the capture probe can bind to a maleimide group on the solid phase) and an amino group or substances having an amino group (the amino group introduced into the capture probe can bind to maleic anhydride on the solid phase). Examples of the affinity substances include streptavidin, biotin, digoxigenin, dinitrophenol, fluorescein, and fluorescein isothiocyanate. In this case, the solid phase coated with another affinity substance having affinity with the affinity substance that the capture probe has can be used. When being used in the form of being free at Step (1), the capture probe may be immobilized to the solid phase after the formation of a hybrid.

Preferably, the probe Y is i) a peptide nucleic acid (PNA) probe (A whole or portion of the probe is PNA. When a portion thereof is PNA, only a nucleotide containing a nucleobase pairing with a modified nucleobase when a heterologous nucleic acid hybrid is formed with the target nucleic acid may be PNA.), ii) a bridged nucleic acid (BNA) probe (In this probe, a whole or portion thereof is BNA. When a portion thereof is BNA, only a nucleotide containing a nucleobase pairing with a modified nucleobase when a heterologous nucleic acid hybrid is formed with the target nucleic acid may be BNA.), or iii) a chimera nucleic acid probe in which a PNA probe and/or BNA probe and another nucleic acid probe described above are linked. In this case, the capture probe is preferably heterologous to both of the target nucleic acid and the probe Y. Since the target nucleic acid is preferably DNA, the capture probe is preferably a modified RNA probe as described above (e.g., a 2'-O-methylated RNA probe).

In a specific embodiment, the "probe Y" or the "capture probe" can be used instead of the "probe X," as mentioned above. Accordingly, embodiments in which the term "probe X" is replaced with "probe Y" or "capture probe" are intended to be included within the scope of the present invention.

At Step (1), the incubation is performed in an appropriate solution on the condition that, when the target nucleic acid is contained in the nucleic acid sample, a hybridization reaction of the probe X (being free or immobilized to the solid phase described below) (or the probe Y and capture probe) and the target nucleic acid in the nucleic acid sample is made possible. As the solution, a buffer solution containing a salt (e.g., sodium citrate) and other ingredients (e.g., a surfactant) can be used, for example. The hybridization temperature is within the range of 15 to 95°C (preferably 25 to 65°C), for example.

When the nucleic acid sample does not contain the target nucleic acid, even when the nucleic acid sample and the probe X (or the probe Y and capture probe) are incubated in the solution, the heterologous nucleic acid hybrid is not formed. In this case, the modified nucleobase cannot be detected at Step (2) described below, but it can be determined that the modified nucleobase is not present in the nucleic acid sample.

When the nucleic acid sample contains the target nucleic acid not containing the modified nucleobase (in other words, the target nucleic acid containing non-modified nucleobases alone), by incubating the nucleic acid sample and the probe X (or the probe Y and capture probe) in the solution, the target nucleic acid not containing the modified nucleobase and the probe X (or the probe Y and capture probe) react with each other, whereby a heterologous nucleic acid hybrid including the target nucleic acid and the probe X (or the probe Y and capture probe) is formed. In this case, the modified nucleobase cannot be detected at Step (2) described below, but it can be determined that the modified nucleobase is not present in the nucleic acid sample (even though the target nucleic acid is present) or, in other words, that a certain nucleobase in the target nucleic acid is not modified.

When the nucleic acid sample contains the target nucleic acid containing the modified nucleobase, by incubating the nucleic acid sample and the probe X (or the probe Y and capture probe) in the solution, the target nucleic acid containing the modified nucleobase and the probe X (or the probe Y and capture probe) react with each other, whereby a heterologous nucleic acid hybrid including the target nucleic acid and the probe X (or the probe Y and capture probe) is formed. In this case, it can be determined that the modified nucleobase is present at Step (2) described below, and the modified nucleobase can also be quantified.

In the present invention, the heterologous nucleic acid hybrid is a hybridization complex having a double-stranded structure of the target nucleic acid and the probe X formed by the hybridization. Alternatively, in the cases where the probe Y and capture probe are used instead of the probe X, the heterologous nucleic acid hybrid is a hybridization complex which includes the target nucleic acid, the probe Y, and the capture probe and has a double-stranded structure of the target nucleic acid and the probe Y formed by the hybridization between the target nucleic acid and the probe Y and a double-stranded structure of the target nucleic acid and the capture probe formed by the hybridization between the target nucleic acid and the capture probe.

In the heterologous nucleic acid hybrid, the number of nucleotide residues of the target nucleic acid and the probe X corresponding to a double-stranded structure part (that is, the lengths of the double-stranded structure parts); or the number of nucleotide residues of the target nucleic acid and the probe Y corresponding to a double-stranded structure part of the target nucleic acid and the probe Y; and the number of nucleotide residues of the target nucleic acid and the capture probe corresponding to a double-stranded structure part of the target nucleic acid and the capture probe; are not limited to a particular number so long as they are long enough to enable hybridization with the target nucleic acid, and may be 10 or more, preferably 15 or more, and more preferably 20 or more, for example. The number of nucleotide residues of the target nucleic acid and the probe X corresponding to a double-stranded structure part; or the number of nucleotide residues of the target nucleic acid and the probe Y corresponding to a double-stranded structure part of the target nucleic acid and the probe Y; and the number of nucleotide residues of the target nucleic acid and the capture probe corresponding to a double-stranded structure part of the target nucleic acid and the capture probe; may also be 100 or less, 80 or less, 60 or less, 50 or less, 40 or less, or 30 or less, for example. A GC content of the double-stranded structure part is not limited to a particular value and may be 10% or more, 20% or more, 30% or more, 40% or more, 50 % or more, or 60% or more, for example. The GC content of the double-stranded structure part may also be 90% or less, 80% or less, or 70% or less, for example. A melting temperature (Tm1 between the target nucleic acid and the probe Y and a melting temperature (Tm2) between the target nucleic acid and the capture probe can be adjusted appropriately in accordance with the lengths of the probe Y and the capture probe (that is, the number of the nucleotide residues). The probe Y and the capture probe may be designed so that the temperature difference between Tm1 and Tm2 will be within the range of 20°C, 15°C, 10°C, or 5°C, for example.

The method according to the present invention may further include preparing a solution containing a nucleic acid sample and the probe X; or a solution containing a nucleic acid sample, the probe Y and capture probe; by adding the probe X, or the probe Y and capture probe to the nucleic acid sample containing the nucleic acid sample. The probe X, or the probe Y and capture probe can be added to the nucleic acid sample as a solid form or as a solution.

When the incubation solution described above is prepared from a nucleic acid sample containing a target nucleic acid containing a modified nucleobase, the concentration of the target nucleic acid in the solution is not limited to a particular value so long as it is detectable by the method according to the present invention and may be 0.01 nM or more, preferably 0.1 nM or more, more preferably 1 nM or more, still more preferably 5 nM or more, and particularly preferably 10 nM or more, for example.
The concentration of the target nucleic acid in the solution may also be 1 M or less, 100 mM or less, 10 mM or less, 1 mM or less, 100 µM or less, 10 µM or less, or 1 µM or less, for example.

The concentration of the probe X in the solution is not limited to a particular value so long as the target nucleic acid is detectable by the method according to the present invention and may be 0.01 nM or more, preferably 0.1 nM or more, more preferably 1 nM or more, still more preferably 5 nM or more, and particularly preferably 10 nM or more, for example. The concentration of the probe X in the solution may also be 1 M or less, 100 mM or less, 10 mM or less, 1 mM or less, 100 µM or less, 10 µM or less, or 1 µM or less, for example. Accordingly, the probe X may be added to the solution so that such a concentration will be achieved.

The concentration of the probe Y in the incubation solution is not limited to a particular value so long as the target nucleic acid is detectable by the method according to the present invention and may be 0.01 nM or more, preferably 0.1 nM or more, more preferably 1 nM or more, still more preferably 5 nM or more, and particularly preferably 10 nM or more, for example. The concentration of the capture probe in the solution may also be 1 M or less, 100 mM or less, 10 mM or less, 1 mM or less, 100 µM or less, 10 µM or less, or 1 µM or less, for example. Accordingly, the probe Y may be added to the solution so that such a concentration will be achieved.

The concentration of the capture probe in the incubation solution is not limited to a particular value so long as the target nucleic acid is detectable by the method according to the present invention and may be 0.01 nM or more, preferably 0.1 nM or more, more preferably 1 nM or more, still more preferably 5 nM or more, and particularly preferably 10 nM or more, for example. The concentration of the capture probe in the solution may also be 1 M or less, 100 mM or less, 10 mM or less, 1 mM or less, 100 µM or less, 10 µM or less, or 1 µM or less, for example. Accordingly, the capture probe may be added to the solution so that such a concentration will be achieved.

The concentration ratio between the probe Y and the capture probe in the incubation solution is not limited to a particular ratio so long as the modified nucleobase in the target nucleic acid can be measured. The concentration ratio (the heterologous guide probe to the capture probe) may be 1:100 to 100:1, 1:50 to 50:1, 1:20 to 20:1, or 1:10 to 10:1, for example. Alternatively, the capture probe may be used in a higher concentration than the probe Y.

In a preferable embodiment, the target nucleic acid may be a target nucleic acid optionally containing two or more modified nucleobases. The number of the modified nucleobases optionally contained in the target nucleic acid is not limited to a particular number so long as it is two or more and is 2 to 30, 2 to 20, 2 to 10, or 2 to 5 (e.g., 2, 3, 4, or 5), for example. When a plurality of modified nucleobases are contained in the target nucleic acid, even when the concentration of the target nucleic acid in the solution used for the hybridization between the target nucleic acid and the probe X is extremely low, it has been confirmed that the modified nucleobases can be measured with high sensitivity. Accordingly, the method according to the present invention can use the probe X (or the probe Y) that is designed so as to hybridize with the target nucleic acid optionally containing two or more modified nucleobases. When the number of nucleobases optionally modified in the target nucleic acid to be measured is determined, such design is made possible.

In a preferable embodiment, the nucleic acid sample is a sample containing a single-stranded target nucleic acid (preferably a target DNA) containing a modified nucleobase. In this case, Step (1) may include performing incubation for a denaturation reaction of the single-stranded target nucleic acid in addition to performing incubation for the hybridization reaction of the single-stranded target nucleic acid, the capture probe, and the guide probe. The incubation for the hybridization reaction can be performed on the hybridization conditions described above, and the incubation for the denaturation reaction can be performed on the conditions of 1 minute to 30 minutes (preferably 2 minutes to 10 minutes) at 70°C to 100°C (preferably 80°C to 98°C), for example.

In another preferable embodiment, the nucleic acid sample is a sample containing a double-stranded target nucleic acid (preferably a target DNA) containing a modified nucleobase. In this case, Step (1) may include performing incubation for dissociation and denaturation reactions of the double-stranded target nucleic acid in addition to performing incubation for the hybridization reaction of the double-stranded target nucleic acid, the capture probe, and the guide probe. The incubation for the hybridization reaction can be performed on the hybridization conditions described above, and the incubation for the dissociation and denaturation reactions can be performed on the conditions of 1 minute to 30 minutes (preferably 2 minutes to 10 minutes) at 70°C to 100°C (preferably 80°C to 98°C), for example.

In still another preferable embodiment, the nucleic acid sample, the capture probe, and the guide probe are incubated in the solution in the presence of a nucleic acid denaturant. The nucleic acid denaturant refers to a substance that has the capability of denaturating a nucleic acid by destroying a higher order structure of the nucleic acid. The concentration of the nucleic acid denaturant in the incubation solution is preferably set so as to increase detection sensitivity for the modified nucleobase in the target nucleic acid (especially, the double-stranded target nucleic acid). The concentration is a concentration exceeding 0.5 M and preferably 1 M or more, for example. The concentration may also be 20 M or less, 10 M or less, 8 M or less, 6 M or less, 4 M or less, 3 M or less, or 2.5 M or less. The nucleic acid denaturant may be a single type or a plurality of types (e.g., two or three types).

Examples of the nucleic acid denaturant includes chaotropic agents and electron donating compounds.

Examples of the chaotropic agents include a guanidinium ion, a barium ion, a calcium ion, a magnesium ion, a thiocyanic acid ion, a perchlorate ion, a nitric acid ion, a bromine ion, an iodide ion, urea, and salts thereof (e.g., metallic salts, inorganic salts, and organic salts). The chaotropic agent is preferably guanidine thiocyanate, guanidine hydrochloride, or urea.

In the present invention, the electron donating compound refers to a compound containing heteroatom, having a nucleic acid denaturation effect and being electron donating. Examples of the heteroatom include a nitrogen atom, an oxygen atom, and a sulfur atom. The electron donating compound is preferably a heterocyclic compound having an electron donating property. Examples of the heterocyclic compound include nonaromatic heterocyclic compounds and compounds having a n electron-excessive aromatic heterocycle (e.g., a five-membered aromatic heterocycle). Examples of the heterocyclic compound having an electron donating property include monocyclic aromatic heterocyclic compounds having an electron donating property and having a five-membered ring structure containing one or two or more heteroatoms in the ring (e.g., pyrrole, pyrazole, and imidazole), fused ring compounds thereof (e.g., indole and benzimidazole), and nonaromatic heterocyclic compounds having an electron donating property and containing one or two or more heteroatoms in the ring (e.g., pyrrolidine, piperidine, and piperazine). The heteroatom is preferably a nitrogen atom.

In a more preferable embodiment, the nucleic acid sample, the capture probe, and the guide probe are incubated in the solution in the presence of both the nucleic acid denaturant and a surfactant. In this case, the nucleic acid denaturant and the concentration of the nucleic acid denaturant in the incubation solution are as described above. The concentration of the surfactant in the incubation solution is preferably set so as to reduce a background value of a detection signal. The concentration is 0.05% (v/v) or more, preferably 0.1% (v/v) or more, and more preferably 0.5% (v/v) or more, for example. The concentration may also be 20% (v/v) or less, 10% (v/v) or less, 8% (v/v) or less, 6% (v/v) or less, 4% (v/v) or less, or 2% (v/v) or less. The surfactant may be a single type or a plurality of types (e.g., two or three types).

Examples of the surfactant include anionic surfactants, cationic surfactants, amphoteric surfactants, and nonionic surfactants.

Examples of the anionic surfactants include hexyl sulfuric acid, octyl sulfuric acid, decyl sulfuric acid, dodecyl sulfuric acid, tetradecyl sulfuric acid, hexadecyl sulfuric acid, dodecyl phosphonic acid, dodecyl benzene sulfonic acid, n-lauroyl sarcosine, n-dodecanoyl sarcosine acid, and salts thereof (e.g., sodium salts).

Examples of the cationic surfactants include quaternary ammonium compounds (e.g., cetyldimethylethylammonium, hexadecyltrimethylammonium, hexadecyltrimethylammonium, and myristyltrimethylammonium), quaternary phosphonium compounds, and salts thereof (e.g., halides).

Examples of the amphoteric surfactants include Zwittergent, ASB-14, 3-N(N,N-dimethyloctylammonio)propane sulfonic acid, 3-n(N,N-dimethyloctylammonio)propane sulfonic acid, 3-(decyldimethylammonio)propane sulfonate acid, N-dodecyl-N,N-dimethyl-3-ammonio-1-propane sulfonic acid, 3-(N,N-dimethylmyristylammonio)propane sulfonic acid, 3-(N,N-dimethylpalmitylammonio)propane sulfonic acid, 3-(N,N-dimethyloctadecylammonio)propane sulfonic acid, and salts thereof.

Examples of the nonionic surfactants include Tween-series surfactants (e.g., Tween-20, Tween-40, Tween-60, and Tween-80), Triton X-series surfactants (e.g., Triton X-100), MEGA-series surfactants (e.g., Mega-8), and NP40.

The modified nucleobase is measured using an antibody against the modified nucleobase having a property of heterologous pairing in a solution containing the heterologous nucleic acid hybrid. In the measurement, although the solution obtained at Step (1) may be used as it is, addition of another solution and/or replacement of the solution with another solution may be performed in order to perform measurement in a solution more suitable for the measurement of the target nucleic acid containing the modified nucleobase by the antibody. The replacement can be performed by adding the solution obtained at Step (1) to a solid phase, immobilizing the heterologous nucleic acid hybrid that can be contained in the solution to the solid phase, then removing the solution from the solid phase, washing it with a cleaning liquid as needed, and adding another solution (e.g., a solution containing the antibody against the modified nucleobase having a property of heterologous pairing) thereto, for example. The solution used in the measurement is not limited to a particular type so long as it is a solution suitable for an antigen-antibody reaction.

The measurement can be performed by immunological methodology. Examples of the immunological methodology include an enzyme immunoassay (EIA) (e.g., direct competitive enzyme-linked immunosorbent assay (ELISA), indirect competitive ELISA, and sandwich ELISA), a radioimmunoassay (RIA), a fluoroimmunoassay (FIA), immunochromatography, a luminescence immunoassay, a spin immunoassay, Western blot, and latex agglutination.

The antibody against the modified nucleobase having a property of heterologous pairing may be a polyclonal antibody or a monoclonal antibody. The antibody against the modified nucleobase having a property of heterologous pairing may be any isotype of immunoglobulin (e.g., IgG, IgM, IgA, IgD, IgE, and IgY). The antibody against the modified nucleobase having a property of heterologous pairing may be a full-length antibody. The full-length antibody refers to an antibody containing a heavy chain and a light chain each containing a variable region and a constant region (e.g., an antibody containing two Fab parts and an Fc part). The antibody against the modified nucleobase having a property of heterologous pairing may also be an antibody fragment derived from the full-length antibody. The antibody fragment is part of the full-length antibody, and examples thereof include F(ab')₂, Fab', Fab, and Fv. The antibody against the modified nucleobase having a property of heterologous pairing may also be a modified antibody such as a single-stranded antibody. The antibody against the modified nucleobase having a property of heterologous pairing may further be an antibody used as a primary antibody in an immunoassay such as ELISA, and in this case, a secondary antibody is used in combination.

In the antibody against a modified nucleobase having a property of heterologous pairing, the term "modified nucleobase having a property of heterologous pairing" refers to a modified nucleobase in the state of being pairing with a complementary nucleobase existing in the probe X. Accordingly, the antibody against a modified nucleobase having a property of heterologous pairing cannot detect a modified nucleobase not forming the heterologous nucleic acid hybrid in the target nucleic acid (e.g., a modified nucleobase in a single-stranded nucleic acid), while the antibody can specifically detect a modified nucleobase forming the heterologous nucleic acid hybrid in the target nucleic acid. Examples of the antibody against the modified nucleobase having a property of heterologous pairing when the target nucleic acid is DNA include 1) antibodies against a pairing unit including a deoxyribonucleoside having a modified nucleobase selected from the group consisting of 2'-deoxy-modified adenosine, 2'-deoxy-modified guanosine, 2'-deoxy-modified cytidine, and 2'-deoxy-modified thymidine and a complementary heterologous nucleic acid thereof (non-deoxyribonucleoside); and 2) antibodies against a pairing unit including a deoxyribonucleotide having a modified nucleobase selected from the group consisting of 2'-deoxy-modified adenosine 5'-phosphate, 2'-deoxy-modified guanosine 5'-phosphate, 2'-deoxy-modified cytidine 5'-phosphate, and 2'-deoxy-modified thymidine 5'-phosphate and a complementary heterologous nucleic acid thereof (non-deoxyribonucleoside). Examples of the antibody against the modified nucleobase having a property of heterologous pairing when the target nucleic acid is RNA include 1') antibodies against a pairing unit including a nucleoside having a modified nucleobase selected from the group consisting of modified adenosine, modified guanosine, modified cytidine, and modified uridine and a complementary heterologous nucleic acid thereof (non-normal RNA); and 2') antibodies against a pairing unit including a nucleotide having a modified nucleobase selected from the group consisting of modified adenosine 5'-phosphate, modified guanosine 5'-phosphate, modified cytidine 5'-phosphate, and modified uridine 5'-phosphate and a complementary heterologous nucleic acid thereof (non-normal RNA).

The antibody against a modified nucleobase having a property of heterologous pairing can be prepared by using the heterologous nucleic acid hybrid including a nucleic acid containing a modified nucleobase and the probe X (definition, examples, and preferable examples are described above) as an antigen (as examples, see the Preparation Examples that will be described later). For example, in vitro methods for preparing monoclonal antibodies, such as a phage display method (Ulman et al, Proc. Natl. Acad. Sci. U.S.A., 90, 1184-89 (1993)) and an ADLib system (WO2004/011644) may be used.

Meanwhile, in the present invention, it has been confirmed that a good antibody against a modified nucleobase having a property of heterologous pairing can efficiently be obtained by using a heterologous nucleic acid hybrid including a modified nucleobase-containing heterologous double strand having a palindromic structure as an antigen (as examples, see the Preparation Examples that will be described later). Accordingly, the antibody against a modified nucleobase having a property of heterologous pairing may be an antibody prepared by using a heterologous nucleic acid hybrid including a modified nucleobase-containing heterologous double strand having a palindromic structure as an antigen. Examples of the modified nucleobase-containing heterologous double strand having a palindromic structure include a heterologous double strand represented by the Formula (I) below. In this case, a heterologous nucleic acid hybrid composed of the target nucleic acid containing the modified nucleobase and the probe X may have a partial structure represented by the Formula (I) or (II) below.

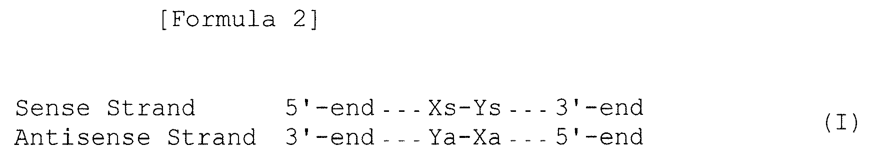

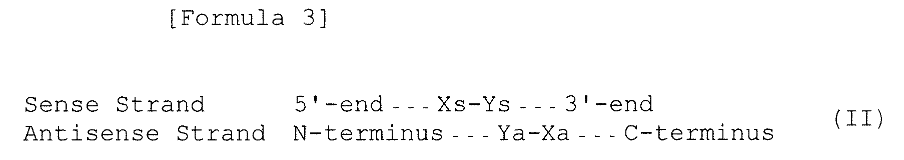

[In Formulae (I) and (II),
Each of "s" and "a" refers to a nucleotide residue in a sense strand and a nucleotide residue in an antisense strand respectively, and the sense strand and the antisense strand are heterologous to each other (see definition and examples of "heterologous" described above).

The nucleobases in nucleotide residues included in Xs and Xa are the same, and refer to residues selected from the group consisting of adenine (A), guanine (G), cytosine (C), thymine (T), and uracil (U).

The nucleobases in nucleotide residues included in Ys and Ya are the same, and refer to residues selected from the group consisting of adenine (A), guanine (G), cytosine (C), thymine (T), and uracil (U).

The nucleobases in nucleotide residues of X and Y are complementary. Xs is pairing with Ya, and Ys is pairing with Xa.

At least one nucleobase (preferably one nucleobase) in a nucleotide residue included in either a sense strand or an antisense strand is modified. Preferably, the nucleotide residue containing a nucleobase capable of pairing with a modified nucleobase has a main chain structure such as the one described above, which is different from the other main chain structure (that a nucleotide residue containing a modified nucleobase has) of the other strand.]

Preferably, the heterologous double strand represented by the Formula (I) above may be a heterologous double strand represented by the Formula (I-1) or (I-2) below. Also, the heterologous double strand represented by the Formula (II) above may be a heterologous double strand represented by the Formula (II-1) or (II-2) below. A heterologous nucleic acid hybrid containing both of a heterologous double strand represented by the Formula (I-1) and that represented by the Formula (I-2) below; or a heterologous nucleic acid hybrid containing both of a heterologous double strand represented by the Formula (II-1) and that represented by the Formula (II-2) below; may be used as an antigen. In these cases, a heterologous nucleic acid hybrid including the target nucleic acid containing the modified nucleobase and the probe X may have a partial structure(s) represented by the Formula (I-1) and/or Formula (I-2) below; and/or Formula (II-1) and/or Formula (II-2) below.

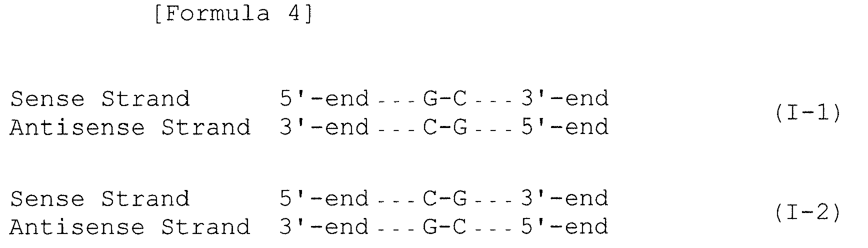

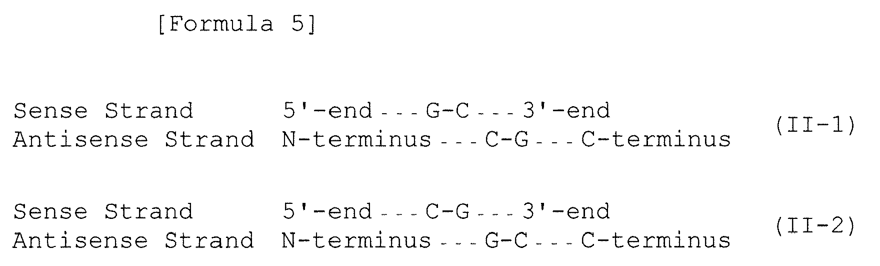

[In Formulae (I-1), (I-2), (II-1), and (II-2),
"G" represents a guanine residue, and "C" represents a cytosine residue.

A cytosine residue included in the sense strand is modified. Preferably, a guanine residue pairing with a modified cytosine residue has a main chain structure such as the one described above, which is different from the main chain structure (that the modified cytosine residue has) of the sense strand.]

The antibody against the modified nucleobase having a property of heterologous pairing may be used while being immobilized or not being immobilized to a solid phase. Examples of the solid phase include supports such as particles (e.g., magnetic particles), membranes (e.g., a nitrocellulose membrane), glass, plastic, and metal, containers such as plates (e.g., a multiwell plate), and devices. The antibody may also be provided in the form of being impregnated into a medium such as filter paper. The antibody against the modified nucleobase having a property of heterologous pairing may be labeled with a labeling substance. Examples of the labeling substance include enzymes (e.g., peroxidase, alkaline phosphatase, luciferase, and β-galactosidase), affinity substances (e.g., streptavidin and biotin), fluorescent substances (e.g., low molecular weight compounds, such as fluorescein, fluorescein isothiocyanate, and rhodamine; and fluorescent proteins, such as a green fluorescent protein and a red fluorescent protein), luminescent substances (e.g., luciferin and aequorin), and radioactive substances (e.g., 3_{H}, ¹⁴C, ³²P, ³⁵S, and ¹²⁵I). Alternatively, when the labeling substance is a protein, the antibody against a modified nucleobase having a property of heterologous pairing may be used as a fusion form with the protein (e.g., enzymes or fluorescent proteins described above). When a secondary antibody is used in the method according to the present invention, the secondary antibody may be labeled with such a labeling substance, or the secondary antibody may be used as a fusion form with the protein (e.g., enzymes or fluorescent proteins described above).

The measurement of the target nucleic acid containing the modified nucleobase by the antibody against the modified nucleobase having a property of heterologous pairing is performed qualitatively or quantitatively, whereby the presence or absence or the amount of the modified nucleobase can be evaluated. In the present invention, the measurement of the target nucleic acid containing the modified nucleobase intends not only the measurement of the modified nucleobase itself but also the measurement of the target nucleic acid containing the modified nucleobase.

The measurement of the presence or absence of the modified nucleobase may be performed as follows, for example:
(2-1) in the solution obtained at Step (1), performing an assay using the antibody against the modified nucleobase having a property of heterologous pairing to measure a signal value;
(2-2) in a solution that does not contain the target nucleic acid containing the modified nucleobase and contains the probe X (or the probe Y and capture probe), performing an assay using the antibody against the modified nucleobase having a property of heterologous pairing to measure a background value; and
(2-3) comparing the signal value with the background value to evaluate the presence or absence of the modified nucleobase.

In the measurement of the target nucleic acid containing the modified nucleobase, the signal value and the background value are values (e.g., absorbance, the degree of fluorescence, the degree of coloration, and radioactivity) that are measured using a label binding to the antibody against the modified nucleobase having a property of heterologous pairing or the secondary antibody (when the secondary antibody is used).

The measurement of the amount of the modified nucleobase may be performed together with the measurement of the background value, for example. Specifically, the measurement of the amount of the modified nucleobase may be performed as follows:
(2-1') in the solution obtained at Step (1), performing an assay using the antibody against the modified nucleobase having a property of heterologous pairing to measure a signal value;
(2-2') in a solution that does not contain the target nucleic acid containing the modified nucleobase and contains the probe X (or the probe Y and capture probe), performing an assay using the antibody against the modified nucleobase having a property of heterologous pairing to measure a background value;
(2-3') correcting the signal value with the background value to obtain a corrected signal value; and
(2-4') based on the corrected signal value, evaluating the amount of the modified nucleobase.

Alternatively, the measurement of the amount of the modified nucleobase may be performed using a standard sample. Specifically, the measurement of the amount of the modified nucleobase may be performed as follows:
(2-1'') in the solution obtained at Step (1), performing an assay using the antibody against the modified nucleobase having a property of heterologous pairing to measure a signal value;
(2-2'') in a solution containing the target nucleic acid containing the modified nucleobase (standard sample) and the probe X (or the probe Y and capture probe), performing an assay using the antibody against the modified nucleobase having a property of heterologous pairing to measure a value for calibration; and
(2-3'') verifying the signal value with the value for calibration to evaluate the amount of the modified nucleobase.

The measurement using the standard sample may be performed in combination with the measurement of the background value.

In a specific embodiment, the method according to the present invention may be performed according to ELISA. For example, when a nucleic acid sample contains the target nucleic acid containing the modified nucleobase, the method according to the present invention according to ELISA may be performed as follows:
(i-1) incubating the nucleic acid sample containing the target nucleic acid containing the modified nucleobase with the probe X (or the probe Y and capture probe) labeled with a first affinity substance in a solution to form a heterologous nucleic acid hybrid including the target nucleic acid and the probe X (or the probe Y and capture probe);
(ii-1) immobilizing the heterologous nucleic acid hybrid to a solid phase treated with a second affinity substance; (iii-1) reacting a primary antibody against the modified nucleobase with the heterologous nucleic acid hybrid immobilized to the solid phase to obtain a primary complex of the primary antibody and the heterologous nucleic acid hybrid;
(iv-1) reacting a secondary antibody labeled with a labeling substance with the primary complex to obtain a secondary complex of the secondary antibody and the primary antibody; and
(v-1) measuring the presence and/or the amount of the formed heterologous nucleic acid hybrid (in other words, the modified nucleobase in the target nucleic acid) by using the labeling substance that the secondary antibody in the secondary complex has.

The first affinity substance and the second affinity substance are used in a combination having mutual affinity (e.g., a combination of biotin and streptavidin). The method according to the present invention may include (i'-1) incubating the nucleic acid sample containing the target nucleic acid containing the modified nucleobase with the probe X immobilized to a solid phase in a solution to form a heterologous nucleic acid hybrid including the target nucleic acid and the probe X in place of Steps (i-1) and (ii-1). In this case, obtaining the probe X immobilized to the solid phase (e.g., adding the probe X labeled with the first affinity substance to the solid phase treated with the second affinity substance) may further be included.
The method according to the present invention may also include washing the solid phase before Step (iii-1). The secondary antibody may be an antibody that recognizes the primary antibody alone (e.g., an antibody that binds to the constant region of the primary antibody) and may also be an antibody that recognizes both the primary antibody in the secondary complex and the primary complex. The method according to the present invention including (i-1) to (v-1) can be performed in accordance with the methodology described in detail in the specification.

The method according to the present invention according to ELISA may also be performed as follows:
(i-2) incubating the nucleic acid sample containing the target nucleic acid containing the modified nucleobase with the probe Y and a capture probe labeled with a first affinity substance in a solution to form a heterologous nucleic acid hybrid including the target nucleic acid, the probe Y, and the capture probe;
(ii-2) immobilizing the heterologous nucleic acid hybrid to a solid phase treated with a second affinity substance; (iii-2) reacting a primary antibody against the modified nucleobase having a property of heterologous pairing with the heterologous nucleic acid hybrid immobilized to the solid phase to obtain a primary complex of the primary antibody and the hybrid;
(iv-2) reacting a secondary antibody labeled with a labeling substance with the primary complex to obtain a secondary complex of the secondary antibody and the primary antibody; and
(v-2) measuring the presence and/or the amount of the formed heterologous nucleic acid hybrid (in other words, the modified nucleobase in the target nucleic acid) by using the labeling substance that the secondary antibody in the secondary complex has.

The first affinity substance and the second affinity substance are used in a combination having mutual affinity (e.g., a combination of biotin and streptavidin). The method according to the present invention may include (i'-2) incubating the nucleic acid sample containing the target nucleic acid containing the modified nucleobase with the probe Y and the capture probe immobilized to a solid phase in a solution to form a heterologous nucleic acid hybrid including the target nucleic acid, the probe Y, and the capture probe in place of Steps (i-2) and (ii-2). In this case, obtaining the capture probe immobilized to the solid phase (e.g., adding the capture probe labeled with the first affinity substance to the solid phase treated with the second affinity substance) may further be included. The method according to the present invention may also include washing the solid phase before Step (iii-2). The secondary antibody may be an antibody that recognizes the primary antibody alone (e.g., an antibody that binds to the constant region of the primary antibody) and may also be an antibody that recognizes both the primary antibody in the secondary complex and the primary complex. In addition, the method according to the present invention including (i-2) to (v-2) can be performed in accordance with the methodology described in detail in the specification.

The present invention also provides a kit for measuring the target nucleic acid containing the modified nucleobase. The kit according to the present invention includes:
(I) a probe X (or a probe Y and capture probe); and
(II) an antibody against a modified nucleobase having a property of heterologous pairing.

The probe X (or the probe Y and capture probe) and the modified nucleobase are as described above. The probe X (or the capture probe) may be labeled with the affinity substance, and the antibody against a modified nucleobase having a property of heterologous pairing may be labeled with the labeling substance, for example. The kit according to the present invention may further contain the above components such as the affinity substance, the labeling substance, the secondary antibody, a detection reagent for the secondary antibody (e.g., when the secondary antibody is labeled with an enzyme, a substrate for the enzyme), and the solid phase. The solid phase may be treated with the affinity substance. The kit according to the present invention may also contain a standard sample of the modified nucleobase or a standard sample of the target nucleic acid containing the modified nucleobase as a solution or as powder.

The kit according to the present invention contains the components in the form of being isolated from each other or in the form of being mixed with each other. In the kit according to the present invention, the components may be provided in the form of being contained in different containers (e.g., a tube and a plate), for example. Alternatively, the kit according to the present invention may be provided in the form of a device. Specifically, all the components may be provided in the form of being contained in a device. Alternatively, a part of the components may be provided in the form of being contained in a device, whereas the rest may be provided in the form of not being contained in the device (e.g., in the form of being contained in different containers). In this case, the components not contained in the device may be used by being injected into the device in the measurement of a target substance.

### EXAMPLES

Although the following describes the present invention with reference to examples, the present invention is not limited to these Examples.

### Example 1: Specific Detection of Modified

Nucleobase Using Antibody against Modified Nucleobase Having a Property of Heterologous Pairing and Heterologous Nucleic Acid Probe Having a Property of Pairing with Modified Nucleobase

The main chain of Sequence 1 is DNA, the sequence thereof is 5'-[C]GTAGGAGGAGGAAG[C]-3' (SEQ ID NO:1: [C] is 5-methylcytosine; the 3'-end is biotin-labeled); that artificially synthesized by Hokkaido System Science Co., Ltd. was used. The main chain of Complementary Chain 1 (nucleic acid probe) is peptide nucleic acid (PNA), the sequence thereof is N-terminus-GCTTCCTCCTCCTACG-C-terminus (SEQ ID NO:2); that artificially synthesized by FASMAC Co., Ltd. was used.

First, Sequence 1 (500 pmol) and Complementary Chain 1 (750 pmol) were reacted at 95°C for 5 minutes, at 60°C for 1 hour, and then at 37°C for 1 hour to obtain a heterologous nucleic acid hybrid (double-stranded nucleic acid including DNA and PNA).

Sequence 2 is the same as Sequence 1 except that the cytosine base was not methylated, and a heterologous nucleic acid hybrid (double-stranded nucleic acid including DNA and PNA) was obtained in the method same as that for Sequence 1, by using Complementary Chain 1 (nucleic acid probe).

The main chain of Sequence 3 is DNA, the sequence thereof is 5'-TTG[C]GCGGCGTC[C]TGTTGACTTC-3' (SEQ ID NO:4; [C] is 5-methylcytosine); the main chain of Complementary Chain 2 (nucleic acid probe) is DNA, the sequence thereof is 5'-GAAGTCAACAGGACGCCGCGCAA-3' (SEQ ID NO:5; the 5'-end is biotin-labeled); those artificially synthesized by Hokkaido System Science Co., Ltd. were used, and a homologous nucleic acid hybrid (double-stranded DNA) was obtained in the method same as that for Sequence 1.

The main chain of Sequence 4 is PNA, the sequence thereof is N-terminus-GGTCTGTGAGTGGTTC-C-terminus (SEQ ID NO:6; the 3'-end is biotin-labeled); that artificially synthesized by FASMAC Co., Ltd. was used.

The main chain of Sequence 5 is DNA, the sequence thereof is 5'-CCAGACACTCACCAAG-3' (SEQ ID NO:7; the 3'-end is biotin-labeled); that artificially synthesized by Hokkaido System Science Co., Ltd. was used.
[Table 1]

**Table 1. Summary of Target Nucleic Acid Used in Example 1**

| Target Nucleic Acid | Main Chain | Nucleotide Sequence | SEQ ID NO |
|---|---|---|---|
| Sequence 1 | DNA | 5'-[C]GT AGG AGG AGG AAG [C]-3' | SEQ ID NO: 1 |
| Sequence 2 | DNA | 5'-CGT AGG AGG AGG AAG C-3' | SEQ ID NO: 3 |
| Sequence 3 | DNA | 5'-TTG [C]GC GGC GTC [C]TG TTG ACT TC-3' | SEQ ID NO: 4 |
| Sequence 4 | PNA | N-terminus-GGT CTG TGA GTG GTT C-C-terminus | SEQ ID NO: 6 |
| Sequence 5 | DNA | 5'-CCA GAC ACT CAC CAA G-3' | SEQ ID NO: 7 |

[Table 2]

**Table 2. Summary of Nucleic Acid Probe Used in Example 1**

| Nucleic Acid Probe | Main Chain | Nucleotide Sequence | SEQ ID NO |
|---|---|---|---|
| Complementary Chain 1 | PNA | N-terminus-GCT TCC TCC TCC TAC G-C-terminus | SEQ ID NO:2 |
| Complementary Chain 2 | DNA | 5'-GAA GTC AAC AGG ACG CCG CGC AA-3' | SEQ ID NO:5 |

First, each of the heterologous nucleic acid hybrid (double-stranded nucleic acid including DNA and PNA), the homologous nucleic acid hybrid (double-stranded DNA), the single-stranded PNA, and the single-stranded DNA prepared above was dissolved in 50 µL of PBS to adjust the concentration to 20 nM, and the total volume was added to a plate (manufactured by Thermo Scientific Inc.) coated with streptavidin, and then was reacted at 37°C for 1 hour to immobilize the nucleic acid on the streptavidin plate. After the reactant was washed with 300 µL of PBS-T three times, the cell culture supernatants obtained in the later-mentioned Preparation Example 1 (5-fold dilution for Clone 10-1-6E, and 10-fold dilution for Clones 10-1-5-4E and 19-3-6H) were added thereto by 50 µL each, and were reacted at 37°C for 1 hour. After the reactant was washed with 300 µL of PBS-T three times, a peroxidase-labeled anti-chicken IgM antibody (prepared in the inventors' company) was added thereto by 50 µL each, and was reacted at 37°C for 1 hour. After the reactant was washed with 300 µL of PBS-T three times, 3,3',5,5'-Tetramethylbenzidine was added thereto by 50 uL each, and was reacted at room temperature for 5 minutes. After that, a 1N sulfuric acid solution was added thereto by 50 µL each, and an absorbance at 450 nm was measured by using a microplate reader (MULTISKAN, manufactured by Thermo Scientific Inc.).

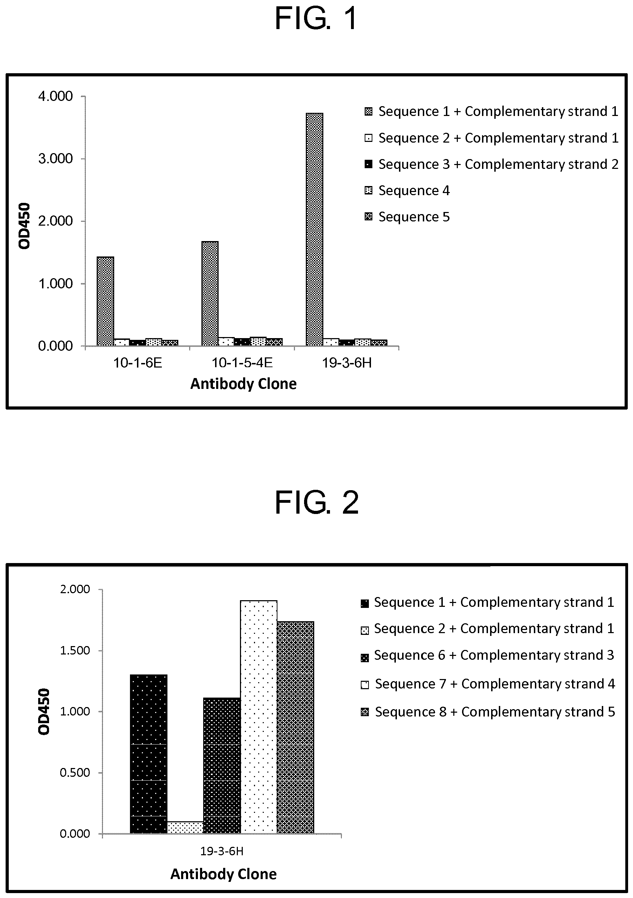

From the result, it has been revealed that the modified nucleobase in the heterologous nucleic acid hybrid can specifically be detected by using the antibody against the modified nucleobase having a property of heterologous pairing and the heterologous nucleic acid probe having a property of pairing with the modified nucleobase (Table 3 and FIG. 1).
[Table 3]

**Table 3. Specific Detection of Modified Nucleobase Using Antibody against Modified Nucleobase Having a Property of Heterologous Pairing and Nucleic Acid Probe Having a Property of Pairing with Modified Nucleobase**

| Antibody Clone | Nucleotide Sequence | Nucleic Acid Probe | OD450 |
|---|---|---|---|
| 10-1-6E | Sequence 1*+Complementary Chain 1 | Heterologous | 1.427 |
| | Sequence 2+Complementary Chain 1 | Heterologous | 0.110 |
| | Sequence 3*+Complementary Chain 2 | Homologous | 0.093 |
| | Sequence 4 | - | 0.119 |
| | Sequence 5 | - | 0.093 |
| 10-1-5-4E | Sequence 1*+Complementary Chain 1 | Heterologous | 1.671 |
| | Sequence 2+Complementary Chain 1 | Heterologous | 0.136 |
| | Sequence 3*+Complementary Chain 2 | Homologous | 0.118 |
| | Sequence 4 | - | 0.139 |
| | Sequence 5 | - | 0.118 |
| 19-3-6H | Sequence 1*+Complementary Chain 1 | Heterologous | 3.724 |
| | Sequence 2+Complementary Chain 1 | Heterologous | 0.120 |
| | Sequence 3*+Complementary Chain 2 | Homologous | 0.095 |
| | Sequence 4 | - | 0.112 |
| | Sequence 5 | - | 0.095 |

| | | | |
|---|---|---|---|
| *Indicating that the target sequences (Sequences 1 and 3) contain modified nucleobases. | | | |

Heterologous: the main chain structure of a nucleic acid probe having a property of pairing with a modified nucleobase is different from that of the target nucleic acid.

Homologous: the main chain structure of a nucleic acid probe having a property of pairing with a modified nucleobase is the same as that of the target nucleic acid.

### Example 2: Peripheral Sequence Independent Detection of Modified Nucleobase Using Antibody against Modified Nucleobase Having a Property of Heterologous Pairing and Heterologous Nucleic Acid Probe Having a Property of Pairing with Modified Nucleobase

The heterologous nucleic acid hybrid including Sequence 1 (DNA) and Complementary Chain 1 (PNA); and the heterologous nucleic acid hybrid including Sequence 2 (DNA) and Complementary Chain 1 (PNA); were prepared in the same method as in Example 1.

The main chain of Sequence 6 is DNA, the sequence thereof is 5'-TAGAA[C]GCTTTG[C]GT-3' (SEQ ID NO:8: [C] is 5-methylcytosine; the 5'-end is biotin-labeled); that artificially synthesized by Hokkaido System Science Co., Ltd. was used. The main chain of Complementary Chain 3 (nucleic acid probe) is PNA, the sequence thereof is N-terminus-ACGCAAAGCGTTCTA-C-terminus (SEQ ID NO:9); that artificially synthesized by FASMAC Co., Ltd. was used.

First, Sequence 6 (2 nmol) and Complementary Chain 3 (4 nmol) were dissolved in 100 µL of a hybridization buffer (4×SSC, 0.08% Tween 20). The solution was subjected to a reaction at 99°C for 5 minutes, at 60°C for 1 hour, and at 37°C for 1 hour to obtain a heterologous nucleic acid hybrid (double-stranded nucleic acid including DNA and PNA).

The main chain of Sequence 7 is DNA, the sequence thereof is 5'-GT[C]GTTTT[C]GG[C]GGC[C]GC-3' (SEQ ID NO:10: [C] is 5-methylcytosine); that artificially synthesized by Hokkaido System Science Co., Ltd. was used. The main chain of Complementary Chain 4 (nucleic acid probe) is PNA, the sequence thereof is N-terminus-GCGGCCGCCGAAAACGAC-C-terminus (SEQ ID NO:11; the N-terminus is biotin-labeled); that artificially synthesized by FASMAC Co., Ltd. was used.

First, Sequence 7 (4 nmol) and Complementary Chain 4 (2 nmol) were dissolved in 100 µL of a hybridization buffer (4×SSC, 0.08% Tween 20). The solution was subjected to a reaction at 99°C for 5 minutes, at 60°C for 1 hour, and at 37°C for 1 hour to obtain a heterologous nucleic acid hybrid (double-stranded nucleic acid including DNA and PNA).

The main chain of Sequence 8 is DNA, the sequence thereof is 5'-GCC[C]GCA[C]GTCCT[C]G[C]GG-3' (SEQ ID NO:12: [C] is 5-methylcytosine; the 5'-end is biotin-labeled); that artificially synthesized by Hokkaido System Science Co., Ltd. was used. The main chain of Complementary Chain 5 (nucleic acid probe) is PNA, the sequence thereof is N-terminus-CCGCGAGGACGTGCGGGC-C-terminus (SEQ ID NO:13); that artificially synthesized by FASMAC Co., Ltd. was used. A heterologous nucleic acid hybrid (double-stranded nucleic acid including DNA and PNA) was obtained in the method same as that for Sequence 6.
[Table 4]

**Table 4. Summary of Target Nucleic Acid Used in Example 2**

| Target Nucleic Acid | Main Chain | Nucleotide Sequence | SEQ ID NO |
|---|---|---|---|
| Sequence 1 | DNA | 5'-[C]GT AGG AGG AGG AAG [C]-3' | SEQ ID NO:1 |
| Sequence 2 | DNA | 5'-CGT AGG AGG AGG AAG C-3' | SEQ ID NO:3 |
| Sequence 6 | DNA | 5'-TAG AA[C] GCT TTG [C]GT-3' | SEQ ID NO:8 |
| Sequence 7 | DNA | 5'-GT[C] GTT TT[C] GG[C] GGC [C]GC-3' | SEQ ID NO:10 |
| Sequence 8 | DNA | 5'-GCC [C]GC A[C]G TCC T[C]G [C]GG-3' | SEQ ID NO:12 |

[Table 5]

**Table 5. Summary of Heterologous Nucleic Acid Probe Having a Property of Pairing with Modified Nucleobase Used in Example 2**

| Probe X | Main Chain | Nucleotide Sequence | SEQ ID NO |
|---|---|---|---|
| Complementary Chain 1 | PNA | N-terminus-GCT TCC TCC TCC TAC G-C-terminus | SEQ ID NO:2 |
| Complementary Chain 3 | PNA | N-terminus-ACG CAA AGC GTT CTA-C-terminus | SEQ ID NO:9 |
| Complementary Chain 4 | PNA | N-terminus-GCG GCC GCC GAA AAC GAC-C-terminus | SEQ ID NO:11 |
| Complementary Chain 5 | PNA | N-terminus-CCG CGA GGA CGT GCG GGC-C-terminus | SEQ ID NO:13 |

First, the heterologous nucleic acid hybrid (double-stranded nucleic acid including DNA and PNA) prepared above was dissolved in 50 µL of PBS to adjust the concentration to 20 nM, and the total volume was added to a plate (manufactured by Thermo Scientific Inc.) coated with streptavidin, and then was reacted at 37°C for 1 hour to immobilize the nucleic acid on the streptavidin plate. After the reactant was washed with 300 µL of PBS-T three times, the cell culture supernatants obtained in the later-mentioned Preparation Example 1 (Clone 19-3-6H, 100-fold dilution) were added thereto by 50 µL each, and were reacted at 37°C for 1 hour. After the reactant was washed with 300 µL of PBS-T three times, a peroxidase-labeled anti-chicken IgM antibody (prepared in the inventors' company) was added thereto by 50 µL each, and was reacted at 37°C for 1 hour. After the reactant was washed with 300 µL of PBS-T three times, 3,3',5,5'-Tetramethylbenzidine was added thereto by 50 µL each, and was reacted at room temperature for 5 minutes. After that, a 1N sulfuric acid solution was added thereto by 50 µL each, and an absorbance at 450 nm was measured by using a microplate reader (MULTISKAN, manufactured by Thermo Scientific Inc.).

From the result, it has been revealed that a modified nucleobase can specifically be recognized by the method according to the present invention, regardless of peripheral sequences of the modified nucleobase in a heterologous nucleic acid hybrid (Table 6 and FIG. 2).
[Table 6]

**Table 6. Peripheral Sequence Independent Detection of Modified Nucleobase Using Antibody against Modified Nucleobase Having a Property of Heterologous Pairing and Heterologous Nucleic Acid Probe Having a Property of Pairing with Modified Nucleobase**

| Antibody Clone | Nucleotide Sequence | OD450 |
|---|---|---|
| 19-3-6H | Sequence 1^{*}+Complementary Chain 1 | 1.300 |
| | Sequence 2+Complementary Chain 1 | 0.100 |
| | Sequence 6^{*}+Complementary Chain 3 | 1.109 |
| | Sequence 7^{*}+Complementary Chain 4 | 1.907 |
| | Sequence 8^{*}+Complementary Chain 5 | 1.735 |

| | | |
|---|---|---|
| *Indicating that the target sequences (Sequences 1 and 6 to 8) contain modified nucleobases. "Sequence 2+Complementary Chain 1": Negative Control (Sequence 2 does not contain any modified nucleobases) | | |

### Example 3: Dose Dependent Detection of Modified

Nucleobase Using Antibody against Modified Nucleobase Having a Property of Heterologous Pairing and Heterologous Nucleic Acid Probe Having a Property of Pairing with Modified Nucleobase

The sequence of the target nucleic acid was Sequence 6 (DNA) described above; that artificially synthesized by Hokkaido System Science Co., Ltd. was used. The sequence of the heterologous nucleic acid probe having a property of pairing with a modified nucleobase was the above-described Complementary Chain 3 that is a peptide nucleic acid (PNA); that artificially synthesized by FASMAC Co., Ltd. was used.

First, the target nucleic acid (500 pmol) and the heterologous nucleic acid probe having a property of pairing with a modified nucleobase (10 pmol) were dissolved in 25 µL of a hybridization buffer (4×SSC, 0.08% Tween 20). The solution was subjected to a reaction at 95°C for 5 minutes, at 60°C for 1 hour, and at 37°C for 1 hour to obtain a heterologous nucleic acid hybrid (double-stranded nucleic acid including DNA and PNA).

Also, a homologous nucleic acid hybrid (double-stranded DNA) was obtained in the same method by using a probe same as that described above as the nucleic acid probe, except that the main chain of the probe was DNA.
[Table 7]

**Table 7. Summary of Target Nucleic Acid Used in Example 3**

| Target Nucleic Acid | Main Chain | Nucleotide Sequence | SEQ ID NO |
|---|---|---|---|
| Sequence 6 | DNA | 5'-TAG AA[C] GCT TTG [C]GT-3' | SEQ ID NO:8 |

[Table 8]

**Table 8. Summary of Guide Probe Used in Example 3**

| Probe X | Main Chain | Nucleotide Sequence | SEQ ID NO |
|---|---|---|---|
| Complementary Chain 3 | PNA | N-terminus-ACG CAA AGC GTT CTA-C-terminus | SEQ ID NO:9 |

First, the heterologous nucleic acid hybrid (2.5 pmol, 0.5 pmol, or 0.1 pmol) prepared above was dissolved in 50 µL of PBS, and the total volume was added to a plate (manufactured by Thermo Scientific Inc.) coated with streptavidin, and then was reacted at 37°C for 1 hour to immobilize the hybrid of the nucleic acids on the streptavidin plate. The homologous nucleic acid hybrid (2.5 pmol, 0.5 pmol, or 0.1 pmol), a single-stranded target nucleic acid not associated with a complementary chain (2.5 pmol, 0.5 pmol, or 0.1 pmol), or a solution not containing the target nucleic acid were also subjected to the same processes. After the reactant was washed with 300 µL of PBS-T three times, the cell culture supernatants obtained in the later-mentioned Preparation Example 1 (Clone 19-3-6H, 100-fold dilution) were added thereto by 50 µL each, and were reacted at 37°C for 1 hour. After the reactant was washed with 300 µL of PBS-T three times, a peroxidase-labeled anti-chicken IgM antibody (prepared in the inventors' company) was added thereto by 50 µL each, and was reacted at 37°C for 1 hour. After the reactant was washed with 300 µL of PBS-T three times, 3,3',5,5'-Tetramethylbenzidine was added thereto by 100 µL each, and was reacted at room temperature under light shielding for 10 minutes. After that, a 2N hydrochloric acid solution was added thereto by 100 µL each, and an absorbance at 450 nm was measured by using a microplate reader (MULTISKAN, manufactured by Thermo Scientific Inc.).

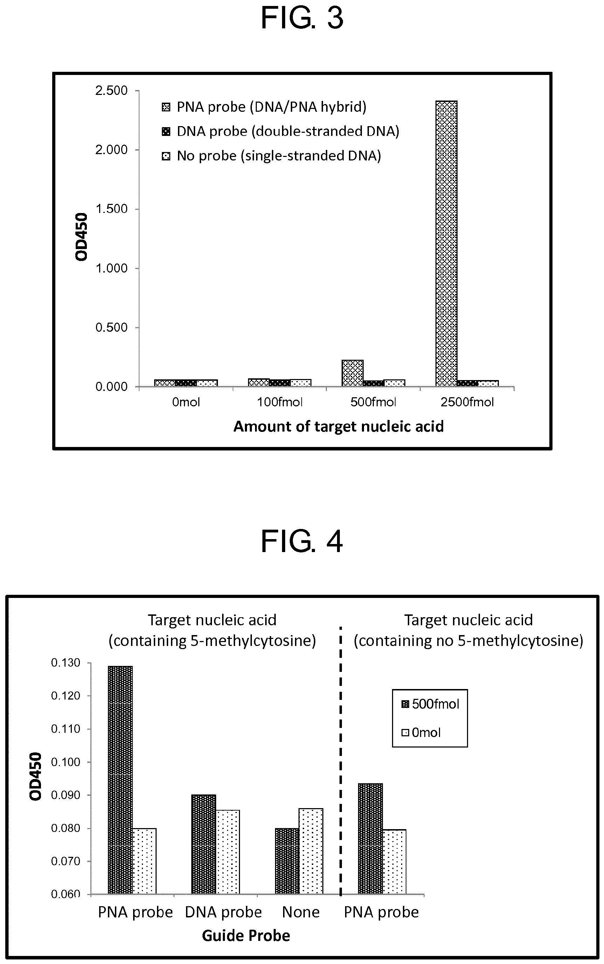

The result demonstrates that the signal was increased depending on the dose of the target nucleic acid only when the heterologous nucleic acid probe having a property of pairing with a modified nucleobase (PNA) was used. Accordingly, it has been revealed that the target nucleic acid can be quantified by the method according to the present invention (Table 9 and FIG. 3).
[Table 9]

**Table 9. Dose Dependent Detection of Modified Nucleobase Using Antibody against Modified Nucleobase Having a Property of Heterologous Pairing and Heterologous Nucleic Acid Probe Having a Property of Pairing with Modified Nucleobase**

| Guide Probe | Amount of Target Nucleic Acid | OD450 |
|---|---|---|
| Heterologous Nucleic Acid Probe Having a Property of Pairing with Modified Nucleobase (PNA) | 2500fmol | 2.410 |
| | 500fmol | 0.226 |
| | 100fmol | 0.067 |
| | 0mol | 0.059 |
| Homologous Nucleic Acid Probe (DNA) | 2500fmol | 0.054 |
| | 500fmol | 0.052 |
| | 100fmol | 0.061 |
| | 0mol | 0.059 |
| None | 2500fmol | 0.053 |
| | 500fmol | 0.060 |
| | 100fmol | 0.063 |
| | 0mol | 0.059 |

Specific Detection of Modified Nucleobase Using Antibody against Modified Nucleobase Having a Property of Heterologous Pairing, Heterologous Guide Probe Pairing with Modified Nucleobase, and Capture Probe

### 4-1) Preparation of Target Nucleic Acid

Polymerase chain reaction (PCR) was used for the preparation of the target nucleic acid sequence. KOD Plus (Product No. KOD-201) manufactured by Toyobo Co., Ltd. was used for an enzyme for PCR. A forward primer: 5'-TAGAACGCTTTGCGTCCCGAC-3' (SEQ ID NO:14) and a reverse primer: 5'-CTGCAGGACCACTCGAGGCTG-3' (SEQ ID NO:15) artificially synthesized by Hokkaido System Science Co., Ltd. were used for two kinds of primers for nucleic acid amplification. A protocol for PCR amplification was 30 cycles of one set including heating at 94°C for 2 minutes, 94°C for 15 seconds, 55°C for 30 seconds, and 68°C for 1 minute.

After performing PCR amplification using a nucleic acid [Sequence: 5'-TAG AAC GCT TTG CGT CCC GAC GCC CGC AGG TCC TCG CGG TGC GCA CCG TTT GCG ACT TGG TGA GTG TCT GGG TCG CCT CGC TCC CGG AAG AGT GCG GAG CTC TCC CTC GGG ACG GTG GCA GCC TCG AGT GGT CCT GCA-3' (SEQ ID NO:16)] artificially synthesized by Hokkaido System Science Co., Ltd. as a template, purification was performed using QIAquick PCR Purification Kit of Qiagen to prepare a 138-bp nucleic acid sequence.

As to the 138-bp nucleic acid sequence prepared as described above, treatment with CpG Methyltransferase (M. SssI) (Product No. EM0821) of Thermo Scientific was performed in order to methylate cytosine of CpG within the nucleic acid sequence. A reaction solution was prepared in accordance with the manufacturer's instructions. The reaction solution was reacted at 37°C for 20 minutes, was further reacted at 65°C for 20 minutes, and was purified using QIAquick Nucleotide Removal Kit of Qiagen to obtain a target nucleic acid.

### 4-2) Detection of Target Nucleic Acid

The capture probe is 5'-UGCAGGACCACUCGAGGCUGCCAC-3' (SEQ ID NO:17; the main chain of the nucleic acid is 2'-O-methylated RNA; the 5'-end is biotin-labeled); that artificially synthesized by Hokkaido System Science Co., Ltd. was used. The sequence of the heterologous guide probe pairing with a modified nucleobase is N-terminus-ACGCAAAGCGTTCTA-C-terminus (SEQ ID NO:18) that is a peptide nucleic acid (PNA); that artificially synthesized by FASMAC Co., Ltd. was used. The target nucleic acids containing 5-methylcytosine used were prepared in Example 4-1).

The same processes were performed by using a probe same as that described above as the homologous guide probe, except that the main chain of the probe was DNA. The homologous guide probe that artificially synthesized by Hokkaido System Science Co., Ltd. was used.

The same processes were performed also in a condition in which the guide probe is not contained.

The same processes were performed also in a condition in which the target nucleic acid was not subjected to the treatment with CpG Methyltransferase (M. SssI) manufactured by Thermo Scientific Inc., described in Example 4, that is, the target nucleic acid sequence in which cytosine of CpG has not been methylated was used.

First, each of the target nucleic acid containing 5-methylcytosine (500 fmol), the capture probe (1 pmol), and the heterologous or homologous guide probe (5 pmol) was dissolved in 50 µL of a hybridization buffer (100 mM Tris-Cl, 1.5 M imidazole, and 50 mM EDTA-2Na). The solution was subjected to a reaction at 95°C for 5 minutes, and at 50°C for 1 hour to form a hybrid of the three, that is, the target nucleic acid, the capture probe, and the heterologous or homologous guide probe. A solution not containing the target nucleic acid was also prepared, and was subjected to the same processes. The total volume of a solution after the hybridization reaction was added to a plate (manufactured by Thermo Scientific Inc.) coated with streptavidin, and then was reacted at 37°C for 1 hour to immobilize the hybrid of the nucleic acids on the streptavidin plate. After the reactant was washed with 300 µL of PBS-T three times, the cell culture supernatants obtained in the later-mentioned Preparation Example 1 (Clone 19-3-6H, 50-fold dilution) were added thereto by 50 µL each, and were reacted at 37°C for 1 hour. After the reactant was washed with 300 µL of PBS-T three times, a peroxidase-labeled anti-chicken IgM antibody (prepared in the inventors' company) was added thereto by 50 µL each, and was reacted at 37°C for 1 hour. After the reactant was washed with 300 µL of PBS-T three times, 3,3',5,5'-Tetramethylbenzidine was added thereto by 100 µL each, and was reacted at room temperature under light shielding for 15 minutes. After that, a 2N hydrochloric acid solution was added thereto by 100 µL each, and an absorbance at 450 nm was measured by using a microplate reader (Arvo, manufactured by PerkinElmer Inc.).

As a result, the method according to the present invention using the antibody against a modified nucleobase having a property of heterologous pairing, the heterologous guide probe pairing with a modified nucleobase, and the capture probe could specifically detect the modified nucleobase (Table 10 and FIG. 4).
[Table 10]

**Table 10. Specific Detection of Modified Nucleobase Using Antibody against Modified Nucleobase Having a Property of Heterologous Pairing, Heterologous Guide Probe Pairing with Modified Nucleobase, and Capture Probe**

| Target Nucleic Acid | Guide Probe | Amount of Target Nucleic Acid | OD450 |
|---|---|---|---|
| Target Nucleic Acid (Containing 5-Methylcytosine) | Heterologous (PNA) | 500fmol | 0.129 |
| | | 0mol | 0.080 |
| | Homologous (DNA) | 500fmol | 0.090 |
| | | 0mol | 0.086 |
| | None | 500fmol | 0.080 |
| | | 0mol | 0.086 |
| Target Nucleic Acid (Not Containing 5-Methylcytosine) | Heterologous (PNA) | 500fmol | 0.094 |
| | | 0mol | 0.080 |

### Example 5: Specific Detection of Modified

Nucleobase Using Antibody against Modified Nucleobase Having a Property of Heterologous Pairing and Heterologous Nucleic Acid Probe Having a Property of Pairing with Modified Nucleobase (BNA probe) (1)

The main chain of Sequence 6 is DNA, the sequence thereof is 5'-TAGAA[C]GCTTTG[C]GT-3' (SEQ ID NO:8: [C] is 5-methylcytosine; the 5'-end is biotin-labeled); that artificially synthesized by Hokkaido System Science Co., Ltd. was used. The main chain of Complementary Chain 6 (nucleic acid probe) is DNA partially including bridged nucleic acid (BNA), the sequence thereof is 5'-ACGCAAAGC(G)TTCTA-3' (SEQ ID NO:25; (G) is a guanine base having a BNA-NC (N-Me) structure); that artificially synthesized by GeneDesign, Inc. was used.

First, Sequence 6 (1 nmol) and Complementary Chain 6 (2 nmol) were reacted at 95°C for 5 minutes, at 42°C for 1 hour, and at 37°C for 1 hour to obtain a heterologous nucleic acid hybrid (double-stranded nucleic acid including DNA and BNA).

Complementary Chain 7 (SEQ ID NO:26) is same as Complementary Chain 6 except that Complementary Chain 7 does not contain BNA, and a homologous nucleic acid hybrid (double-stranded DNA) was obtained by using Sequence 6 in the same method for Complementary Chain 7.
[Table 11]

**Table 11. Summary of Target Nucleic Acid Used in Example 5**

| Target Nucleic Acid | Main Chain | Nucleotide Sequence | SEQ ID NO |
|---|---|---|---|
| Sequence 6 | DNA | 5'-TAG AA[C] GCT TTG [C]GT-3' | SEQ ID NO:8 |

[Table 12]

**Table 12. Summary of Nucleic Acid Probe Used in Example 5**

| Nucleic Acid Probe | Main Chain | Nucleotide Sequence | SEQ ID NO |
|---|---|---|---|
| Complementary Chain 6 | DNA | 5'-ACG CAA AGC (G)TT CTA-3' | SEQ ID NO:25 |
| Complementary Chain 7 | DNA | 5'-ACG CAA AGC GTT CTA-3' | SEQ ID NO:26 |

First, the heterologous nucleic acid hybrid (double-stranded nucleic acid including DNA and BNA) or the homologous nucleic acid hybrid (double-stranded DNA) prepared above was dissolved in 50 µL of PBS to adjust the concentration to 20 nM, and the total volume was added to a plate (manufactured by Thermo Scientific Inc.) coated with streptavidin, and then was reacted at 37°C for 1 hour to immobilize the nucleic acid on the streptavidin plate. After the reactant was washed with 300 µL of PBS-T three times, the cell culture supernatants obtained in the later-mentioned Preparation Example 2 (Clones 20-H2, 20-E3, 20-G5, and 20-H7) were added thereto by 50 µL each, and were reacted at 37°C for 1 hour. After the reactant was washed with 300 µL of PBS-T three times, a peroxidase-labeled anti-chicken IgM antibody (prepared in the inventors' company) was added thereto by 50 µL each, and was reacted at 37°C for 1 hour. After the reactant was washed with 300 µL of PBS-T three times, 3,3',5,5'-Tetramethylbenzidine was added thereto by 50 µL each, and was reacted at room temperature for 5 minutes. After that, a 1N sulfuric acid solution was added thereto by 50 µL each, and an absorbance at 450 nm was measured by using a microplate reader (MULTISKAN, manufactured by Thermo Scientific Inc.).

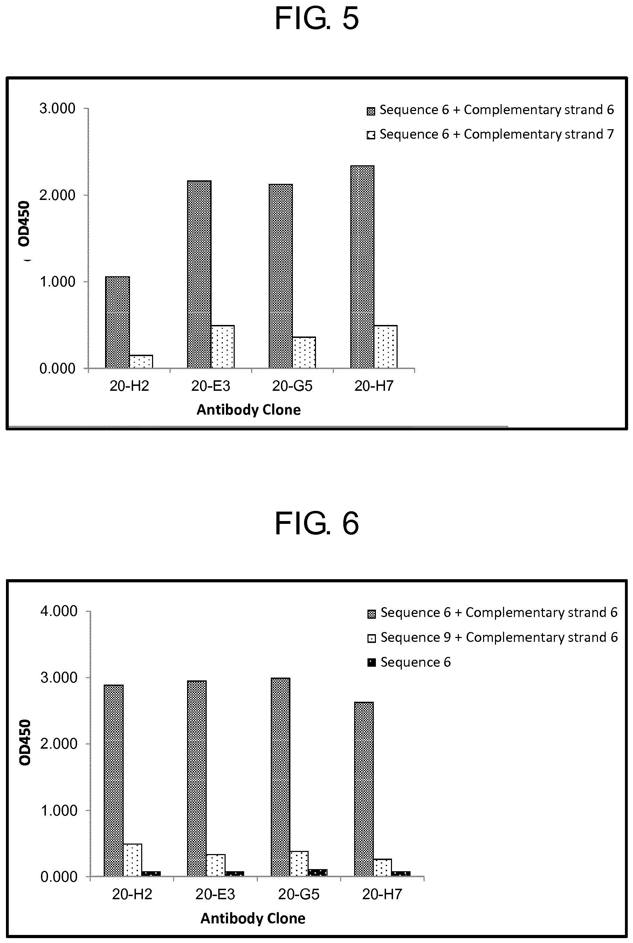

From the result, it has been revealed that the heterologous nucleic acid hybrid can specifically be detected by using the antibody against the modified nucleobase having a property of heterologous pairing and the heterologous nucleic acid probe having a property of pairing with the modified nucleobase (BNA probe) (Table 13 and FIG. 5).
[Table 13]

**Table 13. Specific Detection of Modified Nucleobase Using Antibody against Modified Nucleobase Having a Property of Heterologous Pairing and Nucleic Acid Probe Having a Property of Pairing with Modified Nucleobase (BNA probe) (1)**

| Antibody Clone | Nucleotide Sequence | OD450 |
|---|---|---|
| 20-H2 | Sequence 6+Complementary Chain 6 | 1.054 |
| | Sequence 6+complementary Chain 7 | 0.150 |
| 20-E3 | Sequence 6+Complementary Chain 6 | 2.163 |
| | Sequence 6+Complementary Chain 7 | 0.493 |
| 20-G5 | Sequence 6+Complementary Chain 6 | 2.124 |
| | Sequence 6+Complementary Chain 7 | 0.361 |
| 20-H7 | Sequence 6+Complementary Chain 6 | 2.339 |
| | Sequence 6+Complementary Chain 7 | 0.493 |

### Example 6: Specific Detection of Modified

Nucleobase Using Antibody against Modified Nucleobase Having a Property of Heterologous Pairing and Heterologous Nucleic Acid Probe Having a Property of Pairing with Modified Nucleobase (BNA probe) (2)

The main chain of Sequence 6 is DNA, the sequence thereof is 5'-TAGAA[C]GCTTTG[C]GT-3' (SEQ ID NO:8: [C] is 5-methylcytosine; the 5'-end is biotin-labeled); that artificially synthesized by Hokkaido System Science Co., Ltd. was used. The main chain of Complementary Chain 6 is DNA partially including BNA, the sequence thereof is 5'-ACGCAAAGC(G)TTCTA-3' (SEQ ID NO:25; (G) is a guanine base having a BNA-NC (N-Me) structure); that artificially synthesized by GeneDesign, Inc. was used.

First, Sequence 6 (1 nmol) and Complementary Chain 6 (2 nmol) were reacted at 95°C for 5 minutes, at 42°C for 1 hour, and at 37°C for 1 hour to obtain a heterologous nucleic acid hybrid (double-stranded nucleic acid including DNA containing 5-methylcytosine and BNA).

Sequence 9 (SEQ ID NO:27) is same as Sequence 6 except that the cytosine base is not methylated, and a heterologous nucleic acid hybrid (double-stranded nucleic acid including DNA not containing 5-methylcytosine and BNA) was obtained by using Complementary Chain 6 in the same method for Sequence 6.
[Table 14]

**Table 14. Summary of Target Nucleic Acid Used in Example 6**

| Target Nucleic Acid | Main Chain | Nucleotide Sequence | SEQ ID NO |
|---|---|---|---|
| Sequence 6 | DNA | 5'-TAG AA[C] GCT TTG [C]GT-3' | SEQ ID NO:8 |
| Sequence 9 | DNA | 5'-TAG AAC GCT TTG CGT-3' | SEQ ID NO:27 |

[Table 15]

**Table 15. Summary of Nucleic Acid Probe Used in Example 6**

| Nucleic Acid Probe | Main Chain | Nucleotide Sequence | SEQ ID NO |
|---|---|---|---|
| Complementary Chain 6 | DNA | 5'-ACG CAA AGC (G)TT CTA-3' | SEQ ID NO:25 |

First, each of the two kinds of heterologous nucleic acid hybrids prepared above was dissolved in 50 µL of PBS to adjust the concentration to 20 nM, and the total volume was added to a plate (manufactured by Thermo Scientific Inc.) coated with streptavidin, and then was reacted at 37°C for 1 hour to immobilize the nucleic acid on the streptavidin plate. After the reactant was washed with 300 µL of PBS-T three times, the cell culture supernatants obtained in the later-mentioned Preparation Example 2 (Clones 20-H2, 20-E3, 20-G5, and 20-H7) were added thereto by 50 µL each, and were reacted at 37°C for 1 hour. After the reactant was washed with 300 µL of PBS-T three times, a peroxidase-labeled anti-chicken IgM antibody (prepared in the inventors' company) was added thereto by 50 µL each, and was reacted at 37°C for 1 hour. After the reactant was washed with 300 µL of PBS-T three times, 3,3',5,5'-Tetramethylbenzidine was added thereto by 50 µL each, and was reacted at room temperature for 5 minutes. After that, a 1N sulfuric acid solution was added thereto by 50 µL each, and an absorbance at 450 nm was measured by using a microplate reader (MULTISKAN, manufactured by Thermo Scientific Inc.).

From the result, it has been revealed that the modified nucleobase in the heterologous nucleic acid hybrid can specifically be detected by using the antibody against the modified nucleobase having a property of heterologous pairing and the heterologous nucleic acid probe having a property of pairing with the modified nucleobase (BNA probe) (Table 16 and FIG. 6).
[Table 16]

**Table 16. Specific Detection of Modified Nucleobase Using Antibody against Modified Nucleobase Having a Property of Heterologous Pairing and Nucleic Acid Probe Having a Property of Pairing with Modified Nucleobase (BNA probe) (2)**

| Antibody Clone | Nucleotide Sequence | OD450 |
|---|---|---|
| 20-H2 | Sequence 6+Complementary Chain 6 | 2.886 |
| | Sequence 9+Complementary Chain 6 | 0.492 |
| | Sequence 6 | 0.074 |
| 20-E3 | Sequence 6+Complementary Chain 6 | 2.951 |
| | Sequence 9+Complementary Chain 6 | 0.333 |
| | Sequence 6 | 0.073 |
| 20-G5 | Sequence 6+Complementary Chain 6 | 2.990 |
| | Sequence 9+Complementary Chain 6 | 0.381 |
| | Sequence 6 | 0.107 |
| 20-H7 | Sequence 6+Complementary Chain 6 | 2.627 |
| | Sequence 9+Complementary Chain 6 | 0.261 |
| | Sequence 6 | 0.075 |

### Preparation Example 1: Obtainment of Antibody against Modified Nucleobase Having a Property of Heterologous Pairing (1)

The antibody that recognizes a modified nucleobase in a heterologous nucleic acid hybrid (double-stranded nucleic acid including DNA and PNA) was obtained by the following method utilizing the in vitro antibody obtaining technology [(ADLib (Autonomously Diversifying Library) system: see, for example, WO2004/011644].

### 1) Preparation of Target Antibody-Producing Cell

### 1-1) Diversification of Chicken B Cell Line DT40 Library

The diversification of a chicken B cell line DT40 library was performed by the following method.
a) In a bioreactor tube, 40 mL of the IMDM medium containing 9% FBS and 1% chicken serum [CS (Chicken Serum) +medium] was placed.
b) To the medium, Trichostatin A (TSA) was added so that the concentration thereof becomes 2.5 ng/mL.
c) To the medium, 1.5×10⁷ DT40 cells (a DT40 cell library diversified by utilizing the technique disclosed in WO2004/011644) were added, and were cultured in a 39.5°C CO₂ incubator for one day.

### 1-2) Passage of DT40 Library

The passage of the DT40 library was performed by the following method.
a) The cell suspension one day after the culture was centrifuged at 4°C, 1,000 rpm, for 10 min.
b) After removing the supernatant, cells were resuspended in 10 mL of the CS+ medium.
c) To 950 µL of the CS+ medium, 50 µL of the cell suspension was added for 20-fold dilution, and the mixture was stirred.
d) The number of viable cells was counted.
e) To a fresh bioreactor tube, 40 mL of the CS+ medium was placed.
f) To the medium, 1.5×10⁷ DT40 cells were added, and were cultured in a 39.5°C CO₂ incubator for one day.

Note that the cells were treated with TSA two times before conducting the selection.

### 1-3) Preparation of Antigen

The antigen was prepared according to the method described in Example 1. The heterologous nucleic acid hybrid given below [a double-stranded nucleic acid including Sequence 1 (DNA) and Complementary Chain 1 (PNA)] was used as the antigen.

Nucleic acid (DNA): 5'-CGTAGGAGGAGGAAGC-3' (SEQ ID NO:1)
Complementary Chain 1 (PNA): 3'-GCATCCTCCTCCTTCG-5' (SEQ ID NO:2)
Note) Two cytosine residues of the nucleic acid (DNA) are methylated at the fifth positions.

### 1-4) Preparation of Antigen-Bound Particle

Antigen-bound particles were prepared by the following method.
a) A solution containing 54 pmole of a complex containing DNA containing methylcytosine and PNA was added to magnetic particles on which streptavidin has been immobilized.
b) The mixture was reacted at 4°C for 1 h to allow the complex of DNA and PNA to be solid-phased on the magnetic particles.
c) The magnetic particles were washed with 0.1% BSA/PBS for four times.

### 1-5) Culture of Cell Producing Target Antibody

Cells producing the target antibody were cultured by the following method.
a) To each of two bioreactor tubes, 40 mL of the CS+ medium was placed.
b) To the medium, 1.5×10⁷ cells were added, and were cultured for one day.
c) The cell suspension was centrifuged at 4°C, 1,000 rpm, for 10 min.
d) After removing the supernatant, cells were washed with 1% BSA/PBS two times, and were collected in a 1.5 mL tube.
e) The solution was centrifuged at 4°C, 3,500 rpm, for 5 min to remove the supernatant.
f) The antigen-bound particles prepared in 1-4) above were washed with 1% BSA/PBS four times.
g) The cells and the antigen-bound particles were mixed, and were reacted at 4°C for 30 min.
h) The cells-particles were washed with 1% BSA/PBS five times to remove excess cells.
i) The cells-particles were dispersed with the CS-medium.
j) The cells were placed in a 96 well plate, and were cultured for one week.

### 2) Selection of Cell Producing Target Antibody

The cells producing the target antibody were selected by the antigen solid phase ELISA, based on the difference of coloring between methylcytosine is contained and not contained in the heterologous nucleic acid hybrid (double-stranded nucleic acid including DNA and PNA).

Specifically, the method is as described below.
a) A hybrid between Sequence 1 and Complementary Chain 1 and a hybrid between Sequence 2 and Complementary Chain 1 were prepared in the method described in Example 1.
b) Each of the two kinds of hybrids between DNA and PNA prepared above was dissolved in 50 µL of PBS to adjust the concentration to 20 nM, and the total volume was added to a plate (manufactured by Thermo Scientific Inc.) coated with streptavidin, and then was reacted at 37°C for 1 hour to immobilize the nucleic acid on the streptavidin plate.
c) After the reactant was washed with 300 µL of PBS-T three times, a cell culture supernatant was added thereto by 50 µL each, and was reacted at 37°C for 1 hour.
d) After the reactant was washed with 300 µL of PBS-T three times, a peroxidase-labeled anti-chicken IgM antibody (prepared in the inventors' company) was added thereto by 50 µL each, and was reacted at 37°C for 1 hour.
e) After the reactant was washed with 300 µL of PBS-T three times, 3,3',5,5'-Tetramethylbenzidine was added thereto by 50 µL each, and was reacted at room temperature for 5 minutes.
f) A 1N sulfuric acid solution was added thereto by 50 µL each, and an absorbance at 450 nm was measured by using a microplate reader (MULTISKAN, manufactured by Thermo Scientific Inc.).

As a result, the clones (Clones 10-1-6E, 10-1-5-4E, and 19-3-6H) having remarkable differences of reactivities between methylcytosine is contained and not contained in the heterologous nucleic acid hybrid (double-stranded nucleic acid including DNA and PNA) were successfully obtained.

Preparation Example 2: Obtainment of Antibody against Modified Nucleobase Having a Property of Heterologous Pairing (Double-Stranded Nucleic Acid Including DNA and BNA)

The antibody that recognizes a modified nucleobase in a heterologous nucleic acid hybrid (double-stranded nucleic acid including DNA and BNA) was obtained by the following method utilizing the in vitro antibody obtaining technology (ADLib system) in the same manner as in Preparation Example 1.

### 1) Preparation of Target Antibody-Producing Cell

The target antibody-producing cells were prepared in the same method as in Preparation Example 1, except that the heterologous nucleic acid hybrid (double-stranded nucleic acid including DNA and BNA) prepared in Example 5 was used as the antigen.

### 2) Selection of Cell Producing Target Antibody

The cells producing the target antibody were selected based on the difference of coloring between methylcytosine is contained and not contained in the heterologous nucleic acid hybrid (double-stranded nucleic acid including DNA and BNA) in the same manner as in Preparation Example 1. Specifically, the cells producing the target antibody were selected in the same method as in Preparation Example 1, except that a hybrid between Sequence 6 and Complementary chain 6 and a hybrid between Sequence 9 and Complementary chain 6 were prepared by the method described in Example 6, and were used. The difference of coloring between the heterologous nucleic acid hybrid (double-stranded nucleic acid including DNA and BNA) and the homologous nucleic acid hybrid (double-stranded nucleic acid including DNA and DNA) was also evaluated. Specifically, the cells producing the target antibody were selected in the same method as in Preparation Example 1, except that a hybrid between Sequence 6 and Complementary chain 6 and a hybrid between Sequence 6 and Complementary chain 7 were prepared by the method described in Example 5, and were used.

As a result, the clones (Clones 20-H2, 20-E3, 20-G5, and 20-H7) having remarkable differences of reactivities between methylcytosine is contained and not contained in the heterologous nucleic acid hybrid (double-stranded nucleic acid including DNA and BNA) were successfully obtained.

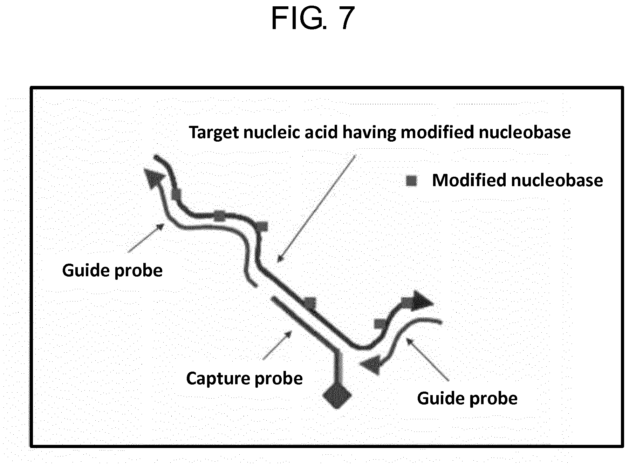

Hereinafter, Reference Examples for the methods in which capture probes and guide probes are used (FIG. 7) will be described.

The inventors of the present invention, through the investigation of a measurement system for the modified nucleobase in the target nucleic acid, have revealed that there is a problem (Specific Problem I) in that detection sensitivity for a modified nucleobase in a double-stranded target nucleic acid is lower (about 1/10) than that for a modified nucleobase in a single-stranded target nucleic acid (Reference Example 1). This is because it is considered that a complementary chain and a capture probe compete against each other for the target nucleic acid containing the modified nucleobase and that hybrid formation efficiency between the target nucleic acid and the capture probe (efficiency of capturing the target nucleic acid to a solid phase) is low (FIG. 8).

In a conventional method for measuring the modified nucleobase in the target nucleic acid using the capture probe, a hybrid including the target nucleic acid and the capture probe is formed (FIG. 9). The conventional method has a potential problem (Specific Problem II) in that a non-hybridized region (a single-stranded region) of the target nucleic acid in this hybrid forms a secondary structure, whereby the modified nucleobase contained in this secondary structure is difficult to be measured (in other words, detection sensitivity is low) (FIG. 9).

An object of the present invention is to increase detection sensitivity for a modified nucleobase in a target nucleic acid.

Another object of the present invention is to increase detection sensitivity for a modified nucleobase in a double-stranded target nucleic acid (that is, to solve Specific Problem I).

Still another object of the present invention is to increase detection sensitivity for a modified nucleobase by avoiding the formation of the secondary structure (that is, to solve Specific Problem II).

Still another object of the present invention is to develop a methodology that can solve these specific problems simultaneously.

Hereinafter, Reference Examples for the methods in which capture probes and guide probes are used so as to solve the objects described above will be described.

### Reference Example 1: Measurement of Modified Nucleobase in Target Nucleic Acid by Capture Probe

### 1-1) Preparation of Target Nucleic Acid

The target nucleic acid was prepared in accordance with the following procedure.

Polymerase chain reaction (PCR) was used for the preparation of the target nucleic acid. KOD Plus (Product No. KOD-201) manufactured by Toyobo Co., Ltd. was used for an enzyme for PCR. A forward primer: 5'-TAGAACGCTTTGCGTCCCGAC-3' (SEQ ID NO:14) and a reverse primer: 5'-CTGCAGGACCACTCGAGGCTG-3' (SEQ ID NO:15) artificially synthesized by Hokkaido System Science Co., Ltd. were used for two kinds of primers for nucleic acid amplification. A protocol for PCR amplification was 30 cycles of one set including heating at 94°C for 2 minutes, 94°C for 15 seconds, 55°C for 30 seconds, and 68°C for 1 minute.

After performing PCR amplification using a nucleic acid (nucleotide sequence: 5'-TAGAACGCTTTGCGTCCCGACGCCCGCAGGTCCTCGCGGTGCGCACCGTTTGCGACTTG GTGAGTGTCTGGGTCGCCTCGCTCCCGGAAGAGTGCGGAGCTCTCCCTCGGGACGGTGG CAGCCTCGAGTGGTCCTGCA-3' (SEQ ID NO:16)) artificially synthesized by Hokkaido System Science Co., Ltd. as a template, purification was performed using QIAquick PCR Purification Kit of Qiagen to prepare a 138-bp nucleic acid.

In order to methylate cytosine of CpG within the 138-bp nucleic acid prepared as described above, treatment with CpG Methyltransferase (M. SssI) (Product No. EM0821) of Thermo Scientific was performed. A reaction solution was prepared in accordance with the manufacturer's instructions. The reaction solution was reacted at 37°C for 20 minutes, was further reacted at 65°C for 20 minutes, and was purified using QIAquick Nucleotide Removal Kit of Qiagen to obtain a target nucleic acid (a methylated double-stranded DNA consisting of the nucleotide sequence of SEQ ID NO:16).

### 1-2) Measurement of Modified Nucleobase in Single-Stranded and Double-Stranded Target Nucleic Acids by Capture Probe

The nucleotide sequence of the capture probe for the target nucleic acid is 5'-UGCAGGACCACUCGAGGCUGCCAC-3' (SEQ ID NO:17) (the main chain of the nucleic acid is 2'-O-methylated RNA, the 5'-end is biotin-labeled); that artificially synthesized by Hokkaido System Science Co., Ltd. was used. As target nucleic acids containing 5-methylcytosine, a single-stranded target nucleic acid (a methylated single-stranded DNA including the nucleotide sequence of SEQ ID NO:16) artificially synthesized by Hokkaido System Science Co., Ltd. and a double-stranded target nucleic acid (a methylated double-stranded DNA including the nucleotide sequence of SEQ ID NO:16) prepared in Reference Example 1-1) were used.

First, the target nucleic acid containing 5-methylcytosine (100 fmol, 10 fmol, 1 fmol, 0.1 fmol, or 0.01 fmol) and the capture probe (5 pmol) were dissolved in 100 µL of a hybridization buffer solution (5× SSC, 0.1% (v/v) Tween 20). The solution was subjected to a reaction [a denaturation reaction (the single-stranded target nucleic acid) or dissociation and denaturation reactions (the double-stranded target nucleic acid)] at 95°C for 5 minutes and was subjected to a hybridization reaction at 37°C for 1 hour to form a hybrid of the target nucleic acid and the capture probe. A solution not containing the target nucleic acid was also prepared, and a similar operation was performed. To the solution after the hybridization reaction, 50 µL of magnetic particles coated with 375 µg/mL of streptavidin (Dynabeads M-280 Streptavidin manufactured by Invitrogen) was added and was reacted at 37°C for 30 minutes to immobilize the nucleic acid hybrid to the magnetic particles. The nucleic acid hybrid immobilized to the magnetic particles was washed with 250 µL of TBS-T three times, and 100 ng/mL of an anti-methylcytosine antibody (Clone33D3 manufactured by Nippon Gene Co., Ltd.) was added thereto by 125 µL each and was reacted at 37°C for 1 hour. The reactant was washed with 250 µL of TBS-T three times, and 250 ng/mL of an alkaline phosphatase-labeled anti-IgG antibody (manufactured by Millipore Corporation) was added thereto by 125 µL each and was reacted at 37°C for 30 minutes. The reactant was washed with 250 µL of TBS-T three times, and a solution of a chemiluminescent substrate AMPPD was added thereto by 110 µL each and was reacted at 37°C for 5 minutes. Thereafter, luminescence counts were measured by a microplate reader (Arvo manufactured by PerkinElmer Inc.).

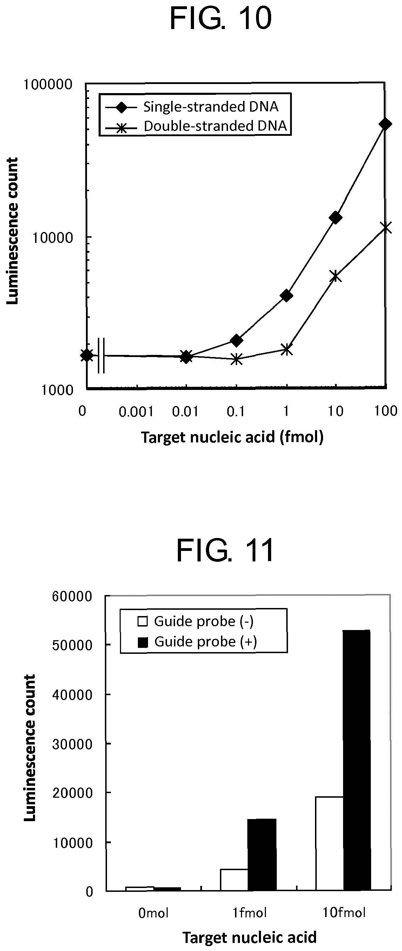

As a result of the measurement, the double-stranded target nucleic acid was lower in the luminescence counts than the single-stranded target nucleic acid, and the double-stranded target nucleic acid was captured to the magnetic particles in an amount only about one-tenth of the single-stranded target nucleic acid (Table 17 and FIG. 10). This can be understood by the fact that the luminescence counts measured when the amount of the double-stranded target nucleic acid was 100 fmol was substantially equal to the luminescence counts measured when the amount of the single-stranded target nucleic acid was 10 fmol, for example (Table 17 and FIG. 10). This fact indicates that a capture rate for the double-stranded target nucleic acid by the capture probe (a hybrid formation rate) is lower than that of the single-stranded target nucleic acid.
[Table 17]

**Table 17. Measurement of Modified Nucleobase in Single-Stranded and Double-Stranded Target Nucleic Acids by Capture Probe**

| | Amount of Target Nucleic Acid | Luminescence Count | S/N |
|---|---|---|---|
| Single-Stranded DNA | 100fmol | 53945 | 32.15 |
| | 10fmol | 13140 | 7.83 |
| | 1fmol | 4079 | 2.43 |
| | 0.1fmol | 2083 | 1.24 |
| | 0.01fmol | 1628 | 0.97 |
| | 0mol | 1678 | |
| Double-Stranded DNA | 100fmol | 11230 | 6.69 |
| | 10fmol | 5497 | 3.28 |
| | 1fmol | 1806 | 1.08 |
| | 0.1fmol | 1584 | 0.94 |
| | 0.01fmol | 1650 | 0.98 |
| | 0mol | 1678 | |

| | | | |
|---|---|---|---|
| S/N: luminescence counts of an amount of the target nucleic acid (fmol)/luminescence counts in the absence (that is, 0 mol) of the target nucleic acid (hereinafter the same will apply unless otherwise noted). | | | |

From the foregoing, Specific Problem I has been revealed.

### Reference Example 2: Measurement of Modified Nucleobase in Single-Stranded Target Nucleic Acid Using Capture Probe and Guide Probe

### 2-1) Measurement Using Capture Probe and Guide Probe

The nucleotide sequence of the capture probe for the target nucleic acid is the nucleotide sequence of SEQ ID NO:17 (the main chain of the nucleic acid is 2'-O-methylated RNA, the 5'-end is biotin-labeled), and the nucleotide sequence of the guide probe is 5'-CCCAGGGAGAGCTCCCACTCTTCCGGAGCAGGCACCCAGACACTCACCAAGTCCAAACG TGCCACCCAGGACCTGCGGCTCGGACCAAAGCTTCTA-3' (SEQ ID NO:19) (Guide Probe 1); those artificially synthesized by Hokkaido System Science Co., Ltd. were used. Guide Probe 1 was designed so as to be able to hybridize with the target nucleic acid in a region different from a region with which the capture probe hybridizes in the target nucleic acid. As the target nucleic acid containing 5-methylcytosine, the single-stranded target nucleic acid (the methylated single-stranded DNA consisting of the nucleotide sequence of SEQ ID NO:16) artificially synthesized by Hokkaido System Science Co., Ltd. was used.

First, the target nucleic acid containing 5-methylcytosine (10 fmol or 1 fmol), the capture probe (5 pmol), and the guide probe (1 pmol) were dissolved in 100 µL of the hybridization buffer solution (5x SSC, 0.1% (v/v) Tween 20). The solution was subjected to a denaturation reaction at 95°C for 5 minutes and was subjected to a hybridization reaction at 37°C for 1 hour to form a hybrid including the target nucleic acid, the capture probe, and the guide probe. A solution not containing the target nucleic acid was also prepared, and a similar operation was performed. To the solution after the hybridization reaction, 50 µL of the magnetic particles coated with 375 µg/mL of streptavidin (Dynabeads M-280 Streptavidin manufactured by Invitrogen) was added and was reacted at 37°C for 30 minutes to immobilize the nucleic acid hybrid to the magnetic particles. The nucleic acid hybrid immobilized to the magnetic particles was washed with 250 µL of TBS-T three times, and 100 ng/mL of the anti-methylcytosine antibody (Clone33D3 manufactured by Nippon Gene Co., Ltd.) was added thereto by 125 µL each and was reacted at 37°C for 1 hour. The reactant was washed with 250 µL of TBS-T three times, and 250 ng/mL of the alkaline phosphatase-labeled anti-IgG antibody (manufactured by Millipore Corporation) was added thereto by 125 µL each and was reacted at 37°C for 30 minutes. The reactant was washed with 250 µL of TBS-T three times, and a solution of the chemiluminescent substrate AMPPD was added thereto by 110 µL each and was reacted at 37°C for 5 minutes. Thereafter, luminescence counts were measured by the microplate reader (Arvo manufactured by PerkinElmer, Inc.).

### 2-2) Measurement Using Capture Probe

### (Conventional Method Not Using Guide Probe)

Tests were carried out by a method similar to that in Reference Example 2-1) except that the guide probe was not added.

### 2-3) Results

The luminescence counts measured using the guide probe remarkably increased compared with the luminescence counts measured without using the guide probe (Table. 18 and FIG. 11).

The fact that the luminescence counts increased by the formation of the hybrid of the single-stranded target nucleic acid and the guide probe indicates that the single-stranded target nucleic acid captured to the solid phase (the magnetic particles) via the capture probe forms the secondary structure in the absence of the guide probe and that it is difficult for the antibody to recognize the modified nucleobase in the secondary structure (FIG. 9).
In other words, it is considered that Specific Problem II was potentially present.

It has been revealed that the guide probe hybridizes with a non-hybridized region (a single-stranded region that can form the secondary structure in the absence of the guide probe) in the hybrid including the single-stranded target nucleic acid and the capture probe, thereby enabling the secondary structure to be loosened, thereby enabling the antibody to efficiently recognize the modified nucleobase (in other words, an increase in detection sensitivity) (refer to Table 18 and FIG. 11). In other words, Specific Problem II has been solved using the guide probe.
[Table 18]

**Table 18. Measurement of Modified Nucleobase in Single-Stranded Target Nucleic Acid Using Capture Probe and Guide Probe**

| Guide Probe | Amount of Target Nucleic Acid | Luminescence Count | S/N |
|---|---|---|---|
| - | 10fmol | 18984 | 22.93 |
| | 1fmol | 4259 | 5.14 |
| | 0mol | 828 | |
| + | 10fmol | 52777 | 75.34 |
| | 1fmol | 14397 | 20.55 |
| | 0mol | 701 | |

| | | | |
|---|---|---|---|
| -: Reference Example 2-2) Without Using Guide Probe +: Reference Example 2-1) Using Guide Probe | | | |

From the foregoing, it has been revealed that the guide probe can increase detection sensitivity for the modified nucleobase in the single-stranded target nucleic acid.

### Reference Example 3: Measurement of Modified Nucleobase in Double-Stranded Target Nucleic Acid Using Capture Probe and Guide Probe

### 3-1) Measurement Using Capture Probe and Guide Probe

Tests were carried out by a method similar to that in Reference Example 2 except that the double-stranded target nucleic acid prepared in Reference Example 1-1) was used as the target nucleic acid containing 5-methylcytosine.

### 3-2) Measurement Using Capture Probe (Without Using Guide Probe)

Tests were carried out by a method similar to that in Reference Example 3-1) except that the guide probe was not added.

### 3-3) Results

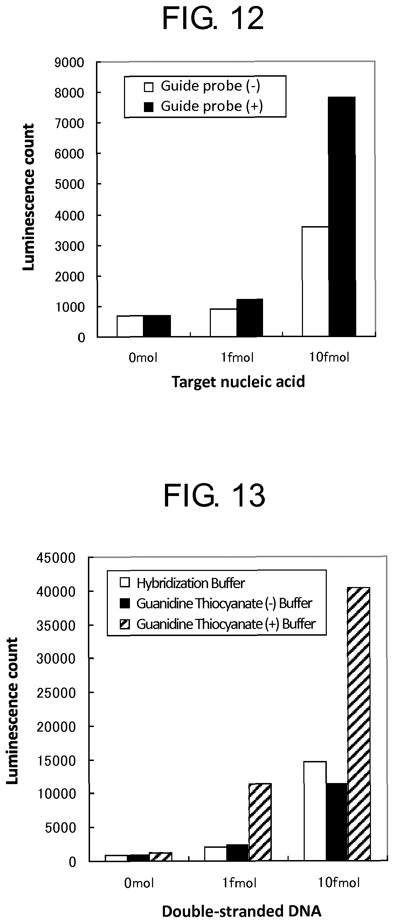

Also in the double-stranded target nucleic acid, the effect of addition of the guide probe was revealed similarly to the single-stranded target nucleic acid (Table 19 and FIG. 12). It is considered that this is because the complementary chain and the capture probe that were competing against each other for the target nucleic acid tended to form the hybrid of the target nucleic acid, the capture probe, and the guide probe through the addition of the guide probe. At the same time, it is considered that this is because even in the double-stranded target nucleic acid the non-hybridized region occurring when forming the hybrid with the capture probe hybridizes with the guide probe, whereby the formation of the secondary structure can be avoided.
[Table 19]

**Table 19. Measurement of Modified Nucleobase in Double-Stranded Target Nucleic Acid Using Capture Probe and Guide Probe**

| Guide Probe | Amount of Target Nucleic Acid | Luminescence Count | S/N |
|---|---|---|---|
| - | 10fmol | 3577 | 5.22 |
| | 1fmol | 917 | 1.34 |
| | 0mol | 686 | |
| + | 10fmol | 7827 | 11.29 |
| | 1fmol | 1215 | 1.75 |
| | 0mol | 693 | |

| | | | |
|---|---|---|---|
| -: Reference Example 3-2) Without Using Guide Probe +: Reference Example 3-1) Using Guide Probe | | | |

From the foregoing, it has been revealed that the guide probe can increase detection sensitivity for the modified nucleobase in the double-stranded target nucleic acid.

### Reference Example 4: Measurement of Modified Nucleobase in Double-Stranded Target Nucleic Acid Using Guide Probe in the Presence of Chaotropic Agent

### 4-1) Measurement Using Guide Probe in the Presence of Chaotropic Agent

The nucleotide sequence of the capture probe for the target nucleic acid is the nucleotide sequence of SEQ ID NO:17 (the main chain of the nucleic acid is 2'-O-methylated RNA, the 5'-end is biotin-labeled), and the nucleotide sequence of the guide probe is the nucleotide sequence of SEQ ID NO:19 (Guide Probe 1); those artificially synthesized by Hokkaido System Science Co., Ltd. were used. As the target nucleic acid containing 5-methylcytosine, the double-stranded target nucleic acid prepared in Reference Example 1-1) was used. As a chaotropic agent, guanidine thiocyanate was used.

First, the double-stranded target nucleic acid containing 5-methylcytosine (10 fmol or 1 fmol), the capture probe (5 pmol), and the guide probe (1 pmol) were dissolved in 100 µL of a guanidine thiocyanate (+) buffer solution (100 mM of Tris-HCl, 4.2 M of guanidine thiocyanate, and 50 mM of EDTA·2Na). The solution was subjected to dissociation and denaturation reactions at 95°C for 5 minutes and was subjected to a hybridization reaction at 37°C for 1 hour to form a hybrid including the target nucleic acid, the capture probe, and the guide probe. A solution not containing the target nucleic acid was also prepared, and a similar operation was performed. To the solution after the hybridization reaction, 50 µL of the magnetic particles coated with 375 µg/mL of streptavidin (Dynabeads M-280 Streptavidin manufactured by Invitrogen) was added and was reacted at 37°C for 30 minutes to immobilize the hybrid to the magnetic particles. The hybrid immobilized to the magnetic particles was washed with 250 µL of TBS-T three times, and 100 ng/mL of the anti-methylcytosine antibody (Clone33D3 manufactured by Nippon Gene Co., Ltd.) was added thereto by 125 µL each and was reacted at 37°C for 1 hour. The reactant was washed with 250 µL of TBS-T three times, and 250 ng/mL of the alkaline phosphatase-labeled anti-IgG antibody (manufactured by Millipore Corporation) was added thereto by 125 µL each and was reacted at 37°C for 30 minutes. The reactant was washed with 250 µL of TBS-T three times, and a solution of the chemiluminescent substrate AMPPD was added thereto by 110 µL each and was reacted at 37°C for 5 minutes. Thereafter, luminescence counts were measured by the microplate reader (Arvo manufactured by PerkinElmer, Inc.).

### 4-2) Measurement Using Guide Probe [in the Absence of Chaotropic Agent (1)]

Tests were carried out by a method similar to that in 3-1) except that the hybridization buffer solution (5x SSC, 0.1% (v/v) Tween 20) was used when the hybrid including the target nucleic acid, the capture probe, and guide probe was formed.

### 4-3) Measurement Using Guide Probe [in the Absence of Chaotropic Agent (2)]

Tests were carried out by a method similar to that in 3-1) except that a guanidine thiocyanate (-) buffer solution (100 mM of Tris-HCl and 50 mM of EDTA·2Na) was used when the hybrid including the target nucleic acid, the capture probe, and guide probe was formed.

### 4-4) Results

When the hybridization reaction was performed on the condition containing the chaotropic agent, the luminescence counts remarkably increased (Table 20 and FIG. 13). This fact indicates that the formation of the hybrid of the double-stranded target nucleic acid and the guide probe is facilitated to increase efficiency of capturing the target nucleic acid to the solid phase (the magnetic particles).
[Table 20]

**Table 20. Measurement of Modified Nucleobase in Double-Stranded Target Nucleic Acid Using Guide Probe in Presence of Chaotropic Agent**

| Buffer Condition | Amount of Target Nucleic Acid | Luminescence Count | S/N |
|---|---|---|---|
| Hybridization Buffer | 10fmol | 14693 | 17.47 |
| | 1fmol | 2048 | 2.44 |
| | 0mol | 841 | |
| Guanidine Thiocyanate (-) | 10fmol | 11445 | 13.99 |
| | 1fmol | 2307 | 2.82 |
| | 0mol | 818 | |
| Guanidine Thiocyanate (+) | 10fmol | 40344 | 32.28 |
| | 1fmol | 11329 | 9.06 |
| | 0mol | 1250 | |

| | | | |
|---|---|---|---|
| Hybridization Buffer: Reference Example 4-2) Absence of Chaotropic Agent (1) Guanidine Thiocyanate (-): Reference Example 4-3) Absence of Chaotropic Agent (2) Guanidine Thiocyanate (+): Reference Example 4-1) Presence of Chaotropic Agent | | | |

From the foregoing, it has been revealed that the guide probe can remarkably increase the detection sensitivity for the modified nucleobase in the double-stranded target nucleic acid in the presence of the chaotropic agent.

### Reference Example 5: Measurement of Modified Nucleobase in Single-Stranded and Double-Stranded Target Nucleic Acids Using Guide Probe in the Presence of Chaotropic Agent

The nucleotide sequence of the capture probe for the target nucleic acid is the nucleotide sequence of SEQ ID NO:17 (the main chain of the nucleic acid is 2'-O-methylated RNA, the 5'-end is biotin-labeled), and the nucleotide sequence of the guide probe is the nucleotide sequence of SEQ ID NO:19 (Guide Probe 1); those artificially synthesized by Hokkaido System Science Co., Ltd. were used. As the target nucleic acids containing 5-methylcytosine, a single-stranded target nucleic acid artificially synthesized by Hokkaido System Science Co., Ltd. and the double-stranded target nucleic acid prepared in Reference Example 1-1) were used. As the chaotropic agent, guanidine thiocyanate was used.

First, the single-stranded or double-stranded target nucleic acid containing 5-methylcytosine (10 fmol, 1 fmol, 0.1 fmol, or 0.01 fmol), the capture probe (5 pmol), and the guide probe (1 pmol) were dissolved in 100 µL of the guanidine thiocyanate (+) buffer solution (100 mM of Tris-HCl, 4.2 M of guanidine thiocyanate, and 50 mM of EDTA·2Na). The solution was subjected to a reaction [a denaturation reaction (the single-stranded target nucleic acid) or dissociation and denaturation reactions (the double-stranded target nucleic acid)] at 95°C for 5 minutes and was subjected to a hybridization reaction at 37°C for 1 hour to form a hybrid including the target nucleic acid, the capture probe, and the guide probe. A solution not containing the target nucleic acid was also prepared, and a similar operation was performed. To the solution after the hybridization reaction, 50 µL of the magnetic particles coated with 375 µg/mL of streptavidin (Dynabeads M-280 Streptavidin manufactured by Invitrogen) was added and was reacted at 37°C for 30 minutes to immobilize the nucleic acid hybrid to the magnetic particles. The nucleic acid hybrid immobilized to the magnetic particles was washed with 250 µL of TBS-T three times, and 100 ng/mL of the anti-methylcytosine antibody (Clone33D3 manufactured by Nippon Gene Co., Ltd.) was added thereto by 125 µL each and was reacted at 37°C for 1 hour. The reactant was washed with 250 µL of TBS-T three times, and 250 ng/mL of the alkaline phosphatase-labeled anti-IgG antibody (manufactured by Millipore Corporation) was added thereto by 125 µL each and was reacted at 37°C for 30 minutes. The reactant was washed with 250 µL of TBS-T three times, and a solution of the chemiluminescent substrate AMPPD was added thereto by 110 µL each and was reacted at 37°C for 5 minutes. Thereafter, luminescence counts were measured by the microplate reader (Arvo manufactured by PerkinElmer, Inc.).

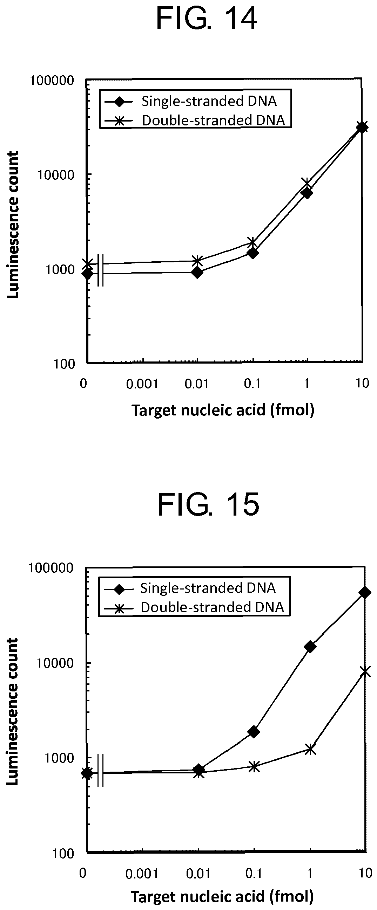

As a result of the measurement, surprisingly, substantially equal luminescence counts were obtained for the single-stranded target nucleic acid and the double-stranded target nucleic acid (Table 21 and FIG. 14). This fact indicates that the guide probe can increase the detection sensitivity for the modified nucleobase in the double-stranded target nucleic acid to be substantially equal to that for the modified nucleobase in the single-stranded target nucleic acid in the presence of the chaotropic agent.
[Table 21]

**Table 21. Measurement of Modified Nucleobase in Single-Stranded and Double-Stranded Target Nucleic Acids Using Guide Probe in Presence of Chaotropic Agent**

| Buffer Condition | Target Nucleic Acid | Amount of Target Nucleic Acid | Luminescence Count | S/N |
|---|---|---|---|---|
| Guanidine Thiocyanate (+) | Single-Stranded DNA | 10fmol | 30660 | 34.82 |
| | | 1fmol | 6338 | 7.20 |
| | | 0.1fmol | 1461 | 1.66 |
| | | 0.01fmol | 906 | 1.03 |
| | | 0mol | 881 | |
| | Double-Stranded DNA | 10fmol | 31156 | 27.53 |
| | | 1fmol | 7948 | 7.02 |
| | | 0.1fmol | 1891 | 1.67 |
| | | 0.01fmol | 1193 | 1.05 |
| | | 0mol | 1132 | |

From the foregoing, it has been revealed that the guide probe can measure the modified nucleobase in the target nucleic acid with high sensitivity regardless of the number of the strand of the target nucleic acid in the presence of the chaotropic agent.

### Reference Example 6: Measurement of Modified Nucleobase in Single-Stranded and Double-Stranded Target Nucleic Acids Using Capture Probe and Guide Probe

Tests were carried out by a method similar to that in Reference Example 5 except that the hybridization buffer solution (5× SSC, 0.1% (v/v) Tween 20) was used in place of the guanidine thiocyanate (+) buffer solution when the hybrid including the target nucleic acid, the capture probe, and the guide probe was formed.

As a result of the measurement, although some increase in the detection sensitivity for the modified nucleobase in the double-stranded target nucleic acid was revealed on the conditions using the hybridization buffer solution (that is, use of the guide probe alone) (the difference was not as much as that revealed in Reference Example 1), the detection sensitivity for the modified nucleobase in the double-stranded target nucleic acid fell short of that for the modified nucleobase in the single-stranded target nucleic acid (Table 22 and FIG. 15). In other words, it has been proved that the guide probe can increase the detection sensitivity for the modified nucleobase in the double-stranded target nucleic acid to be substantially equal to that for the modified nucleobase in the single-stranded target nucleic acid in the presence of the chaotropic agent.
[Table 22]

**Table 22. Measurement of Modified Nucleobase in Single-Stranded and Double-Stranded Target Nucleic Acids Using Guide Probe**

| Buffer Condition | Target Nucleic Acid | Amount of Target Nucleic Acid | Luminescence Count | S/N |
|---|---|---|---|---|
| Hybridization Buffer | Single-Stranded DNA | 10fmol | 52777 | 75.34 |
| | | 1fmol | 14397 | 20.55 |
| | | 0.1fmol | 1863 | 2.66 |
| | | 0.01fmol | 751 | 1.07 |
| | | 0mol | 701 | |
| | Double-Stranded DNA | 10fmol | 7827 | 11.29 |
| | | 1fmol | 1215 | 1.75 |
| | | 0.1fmol | 789 | 1.14 |
| | | 0.01fmol | 695 | 1.00 |
| | | 0mol | 693 | |

### Reference Example 7: Measurement of Modified Nucleobase Using Guide Probe in the Presence of Nucleic Acid Denaturant

Tests were carried out by a method similar to that in Reference Example 4-1) except that the buffer solution (100 mM of Tris-HCl and 50 mM of EDTA·2Na) containing no nucleic acid denaturant, 4.2 M of guanidine thiocyanate, 2.7 M of imidazole, or 4 M of urea was used when the hybrid including the target nucleic acid, the capture probe, and the guide probe was formed.

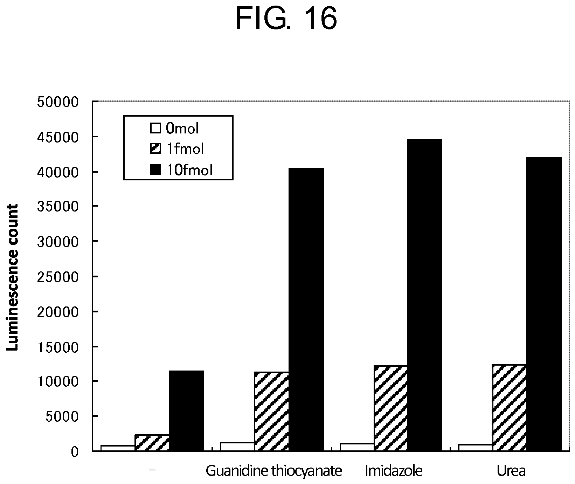

As a result of the measurement, the nucleic acid denaturants other than guanidine thiocyanate also produced luminescence counts equal to those of guanidine thiocyanate (Table 23 and FIG. 16). This fact indicates that the guide probe can increase the detection sensitivity for the modified nucleobase in the target nucleic acid in the presence of the nucleic acid denaturant.
[Table 23]

**Table 23. Measurement of Modified Nucleobase Using Guide Probe in Presence of Nucleic Acid Denaturant**

| Denaturant | Amount of Target Nucleic Acid | Luminescence Count | S/N |
|---|---|---|---|
| - | 10fmol | 11445 | 14.0 |
| | 1fmol | 2307 | 2.8 |
| | 0mol | 818 | |
| Guanidine Thiocyanate | 10fmol | 40344 | 32.3 |
| | 1fmol | 11329 | 9.1 |
| | 0mol | 1250 | |
| Imidazole | 10fmol | 44460 | 44.1 |
| | 1fmol | 12171 | 12.1 |
| | 0mol | 1009 | |
| Urea | 10fmol | 41962 | 49.8 |
| | 1fmol | 12298 | 14.6 |
| | 0mol | 842 | |

| | | | |
|---|---|---|---|
| - : Without Denaturant | | | |

From the foregoing, it has been revealed that the guide probe can measure the modified nucleobase in the target nucleic acid with high sensitivity in the presence of the nucleic acid denaturant.

### Reference Example 8: Inhibition of Formation of Secondary Structure in Site Containing Modified Nucleobase by Guide Probe

The nucleotide sequence of the capture probe for the target nucleic acid is the nucleotide sequence of SEQ ID NO:17 (the main chain of the nucleic acid is 2'-O-methylated RNA, the 5'-end is biotin-labeled), and the nucleotide sequence of the guide probe is any of the nucleotide sequences listed in Table 24; those artificially synthesized by Hokkaido System Science Co., Ltd. were used. As the target nucleic acid containing 5-methylcitosine, the double-stranded target nucleic acid prepared in Reference Example 1-1) was used.

Tests were carried out by a method similar to that in Reference Example 4-1) except that none of the guide probes with the sequences listed in Table 24 was added or one, two, or three thereof were added by 10 pmol each.

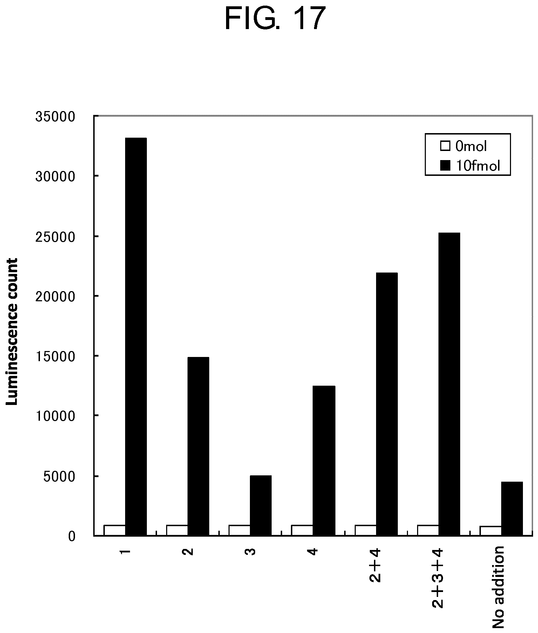

As a result of the measurement, although increases in the luminescence counts were revealed when the guide probes (that is, Guide Probes 1, 2, and 4) having complementarity with a site containing the modified nucleobase in the target nucleic acid were added, no increase in the luminescence counts was revealed when the guide probe (that is, Guide Probe 3) having complementarity with a site not containing the modified nucleobase in the target nucleic acid was added (Table 25 and FIG. 17). Accordingly, it has been demonstrated that inhibition of the formation of the secondary structure in the site containing the modified nucleobase by the guide probe is important for increasing detection sensitivity.
[Table 24]

**Table 24. Nucleotide Sequence of Guide Probe 1 to 4**

| Guide Probe | Nucleotide Sequence (SEQ ID NO) |
|---|---|
| 1 | 5′-CCC AGG GAG AGC TCC CAC TCT TCC GGA GCA GGC ACC CAG ACA CTC ACC AAG TCC AAA CGT GCC ACC CAG GAC CTG CGG CTC GGA CCA AAG CTT CTA-3′ (SEQ ID NO:19) |
| 2 | 5'-TCC CAG GGA GAG CTC CCA CTC TTC CGG AGC AGG C-3' (SEQ ID NO:20) |
| 3 | 5'-ACC CAG ACA CTC ACC AAG TC-3' (SEQ ID NO:21) |
| 4 | 5'-CAA ACG TGC CAC CCA GGA CCT GCG GCT CGG ACC AAA GC-3' (SEQ ID NO:22) |

[Table 25]

**Table 25. Inhibition of Formation of Secondary Structure in Site Containing Modified Nucleobase by Guide Probe**

| Guide Probe | Amount of Target Nucleic Acid | Luminescence Count | S/N |
|---|---|---|---|
| 1 | 10fmol | 33154 | 38.2 |
| | 0mol | 867 | |
| 2 | 10fmol | 14856 | 18.2 |
| | 0mol | 816 | |
| 3 | 10fmol | 4934 | 5.5 |
| | 0mol | 892 | |
| 4 | 10fmol | 12436 | 14.2 |
| | 0mol | 876 | |
| 2+4 | 10fmol | 21898 | 25.1 |
| | 0mol | 874 | |
| 2+3+4 | 10fmol | 25245 | 28.8 |
| | 0mol | 878 | |
| Absence | 10fmol | 4463 | 5.5 |
| | 0mol | 813 | |

| | | | |
|---|---|---|---|
| 2+4: Guide Probes 2 and 4 are added. 2+3+4: Guide Probes 2, 3, and 4 are added. | | | |

### Reference Example 9: Investigation of Concentration of Nucleic Acid Denaturant

Tests were carried out by a method similar to that in Reference Example 4-1) except that the concentration of guanidine thiocyanate contained in the buffer solution (100 mM of Tris-HCl, guanidine thiocyanate, and 50 mM of EDTA·2Na) for forming the hybrid of the target nucleic acid containing 5-methylcytosine (10 fmol or 1 fmol), the capture probe (5 pmol), and Guide Probe 1 (1 pmol) was set to any of the concentrations listed in Table 26. For a guanidine thiocyanate (-) buffer solution (that is, 0 M), tests were carried out similarly.

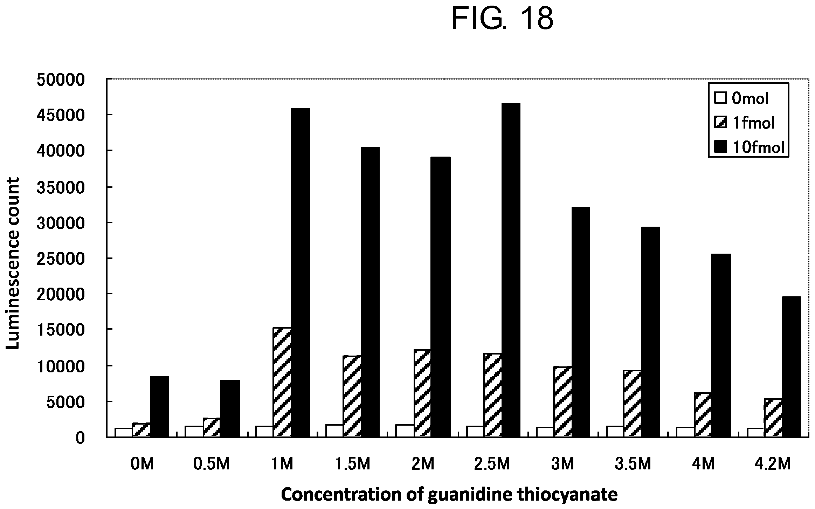

As a result of the measurement, it has been revealed that guanidine thiocyanate contained in the buffer solution in the range of 1 M to 2.5 M is the most effective (Table 26 and FIG. 18).
[Table 26]

**Table 26. Effect of Nucleic Acid Denaturant at Various Concentrations**

| Concentration of Guanidine Thiocyanate | Amount of Target Nucleic Acid | Luminescence Count | S/N |
|---|---|---|---|
| 0M | 10fmol | 8378 | 7.3 |
| | 1fmol | 1862 | 1.6 |
| | 0mol | 1140 | |
| 0.5M | 10fmol | 7862 | 5.4 |
| | 1fmol | 2599 | 1.8 |
| | 0mol | 1463 | |
| 1M | 10fmol | 45811 | 28.3 |
| | 1fmol | 15247 | 9.4 |
| | 0mol | 1621 | |
| 1.5M | 10fmol | 40450 | 24.0 |
| | 1fmol | 11275 | 6.7 |
| | 0mol | 1689 | |
| 2M | 10fmol | 39062 | 22.8 |
| | 1fmol | 12092 | 7.1 |
| | 0mol | 1713 | |
| 2.5M | 10fmol | 46560 | 29.7 |
| | 1fmol | 11571 | 7.4 |
| | 0mol | 1566 | |
| 3M | 10fmol | 32029 | 22.8 |
| | 1fmol | 9809 | 7.0 |
| | 0mol | 1404 | |
| 3.5M | 10fmol | 29208 | 19.8 |
| | 1fmol | 9316 | 6.3 |
| | 0mol | 1477 | |
| 4M | 10fmol | 25430 | 19.0 |
| | 1fmol | 6127 | 4.6 |
| | 0mol | 1340 | |
| 4.2M | 10fmol | 19587 | 15.6 |
| | 1fmol | 5322 | 4.2 |
| | 0mol | 1253 | |

### Reference Example 10: Investigation of Main Chain of Guide Probe

The nucleotide sequence of the capture probe for the target nucleic acid is the nucleotide sequence of SEQ ID NO:17 (the main chain of the nucleic acid is 2'-O-methylated RNA, the 5'-end is biotin-labeled); that artificially synthesized by Hokkaido System Science Co., Ltd. was used. The nucleotide sequence of the guide probe is any of the nucleotide sequences listed in Table 27; Guide Probes 2 and 4 having DNA as the main chain of the nucleic acid were used, whereas Guide Probes 5 and 6 having 2'-O-methylated RNA or RNA as the main chain of the nucleic acid were used. Although Guide Probes 5 and 6 have the sequences equal to those of Guide Probes 2 and 4, respectively, their main chain of the nucleic acid is 2'-O-methylated RNA or RNA, and the guide probes in which the thymine base (T) was changed to the uracil base (U) were used. The guide probes artificially synthesized by Hokkaido System Science Co., Ltd. were used. As the target nucleic acid containing 5-methylcytosine, the double-stranded target nucleic acid prepared in Reference Example 1-1) was used.

Tests were carried out by a method similar to that in Reference Example 4-1) except that none of the guide probes with the sequences listed in Table 27 was added or that one or two thereof were added by 1 pmol each.

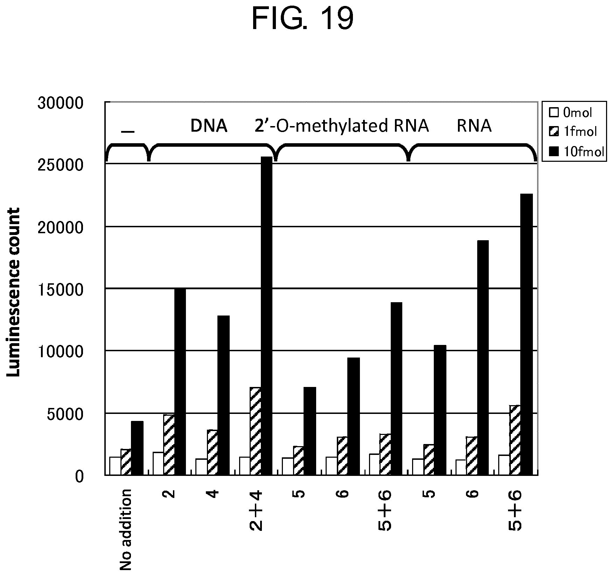

Even when the main chain of the nucleic acid used as the guide probe varied from DNA, RNA, to 2'-O-methylated RNA, increases in the luminescence counts were revealed compared with a case in which the guide probe was absent (Table 28 and FIG. 19). This fact indicates that the guide probe functions as a guide probe regardless of its main chain structure. It has also been revealed that DNA as the main chain of the guide probe is the most effective (Table 28 and FIG. 19).
[Table 27]

**Table 27. Nucleotide Sequence of Guide Probe 2 and 4 to 6**

| Guide Probe | Nucleotide Sequence (SEQ ID NO) |
|---|---|
| 2 | 5'-TCC CAG GGA GAG CTC CCA CTC TTC CGG AGC AGG C-3' (SEQ ID NO:20) |
| 4 | 5'-CAA ACG TGC CAC CCA GGA CCT GCG GCT CGG ACC AAA GC-3' (SEQ ID NO:22) |
| 5 | 5'-UCC CAG GGA GAG CUC CCA CUC UUC CGG AGC AGG C-3' (SEQ ID NO:23) |
| 6 | 5'-CAA ACG UGC CAC CCA GGA CCU GCG GCU CGG ACC AAA GC-3' (SEQ ID NO:24) |

| | |
|---|---|
| Guide Probe 5 is same as Guide Probe 2 except that having U instead of T in the sequence. Guide Probe 6 is same as Guide Probe 4 except that having U instead of T in the sequence. | |

[Table 28]

**Table 28. Investigation of Main Chain of Guide Probe**

| Guide Probe | | Amount of Target Nucleic Acid | Luminescence Count | S/N |
|---|---|---|---|---|
| Nucleic Acid Species | Guide Probe | | | |
| DNA | 2 | 10fmol | 14919 | 8.1 |
| | | 1fmol | 4797 | 2.6 |
| | | 0mol | 1843 | |
| | 4 | 10fmol | 12771 | 9.8 |
| | | 1fmol | 3635 | 2.8 |
| | | 0mol | 1305 | |
| | 2+4 | 10fmol | 25559 | 17.7 |
| | | 1fmol | 7026 | 4.9 |
| | | 0mol | 1448 | |
| 2'-O-methylated RNA | 5 | 10fmol | 7067 | 5.1 |
| | | 1fmol | 2319 | 1.7 |
| | | 0mol | 1387 | |
| | 6 | 10fmol | 9402 | 6.6 |
| | | 1fmol | 3085 | 2.2 |
| | | 0mol | 1418 | |
| | 5+6 | 10fmol | 13885 | 8.1 |
| | | 1fmol | 3263 | 1.9 |
| | | 0mol | 1720 | |
| RNA | 5 | 10fmol | 10433 | 8.2 |
| | | 1fmol | 2483 | 2.0 |
| | | 0mol | 1269 | |
| | 6 | 10fmol | 18815 | 15.2 |
| | | 1fmol | 3057 | 2.5 |
| | | 0mol | 1242 | |
| | 5+6 | 10fmol | 22544 | 14.4 |
| | | 1fmol | 5608 | 3.6 |
| | | 0mol | 1570 | |
| Absence | | 10fmol | 4297 | 3.0 |
| | | 1fmol | 2096 | 1.4 |
| | | 0mol | 1446 | |

| | | | | |
|---|---|---|---|---|
| Guide Probe 2+4: Guide Probes 2 and 4 are added. Guide Probe 5+6: Guide Probes 5 and 6 are added. | | | | |

### Reference Example 11: Measurement of Modified Nucleobase Using Guide Probe in the Presence of Nucleic Acid Denaturant or Non-Nucleic Acid Denaturant

The nucleotide sequence of the capture probe for the target nucleic acid is the nucleotide sequence of SEQ ID NO:17 (the main chain of the nucleic acid is 2'-O-methylated RNA, the 5'-end is biotin-labeled), and the nucleotide sequence of the guide probe is the nucleotide sequence of SEQ ID NO:19 (Guide Probe 1); those artificially synthesized by Hokkaido System Science Co., Ltd. were used. As the target nucleic acid containing 5-methylcytosine, the double-stranded target nucleic acid prepared in Reference Example 1-1) was used.

First, the target nucleic acid containing 5-methylcytosine (10 fmol or 1 fmol), the capture probe (1 pmol), and the guide probe (1 pmol) were dissolved in 100 µL of the buffer solution (100 mM of Tris-HCl and 50 mM of EDTA·2Na). In the buffer solution, a similar solution was prepared using a buffer solution containing 1.5 M of guanidine thiocyanate, 1.5 M of imidazole, 1.5 M of pyrazole, 1.5 M of urea, 1% (v/v) of Tween 20, or 1% (v/v) of sodium lauryl sulfate. The solution was subjected to dissociation and denaturation reactions at 95°C for 5 minutes and was subjected to a hybridization reaction at 37°C for 1 hour to form a hybrid including the target nucleic acid, the capture probe, and the guide probe. A solution not containing the target nucleic acid was also prepared, and a similar operation was performed. To the solution after the hybridization reaction, 50 µL of the magnetic particles coated with 375 µg/mL of streptavidin (Dynabeads M-280 Streptavidin manufactured by Invitrogen) was added and was reacted at 37°C for 30 minutes to immobilize the nucleic acid hybrid to the magnetic particles. The nucleic acid hybrid immobilized to the magnetic particles was washed with 250 µL of TBS-T three times, and 100 ng/mL of the anti-methylcytosine antibody (Clone33D3 manufactured by Nippon Gene Co., Ltd.) was added thereto by 125 µL each and was reacted at 37°C for 1 hour. The reactant was washed with 250 µL of TBS-T three times, and 250 ng/mL of the alkaline phosphatase-labeled anti-IgG antibody (manufactured by Millipore Corporation) was added thereto by 125 µL each and was reacted at 37°C for 30 minutes. The reactant was washed with 250 µL of TBS-T three times, and a solution of the chemiluminescent substrate AMPPD was added thereto by 110 µL each and was reacted at 37°C for 5 minutes. Thereafter, luminescence counts were measured by the microplate reader (Arvo manufactured by PerkinElmer, Inc.).

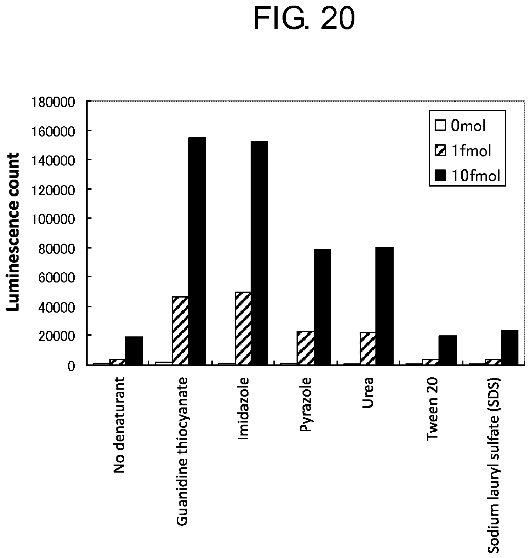

As a result of the measurement, the surfactants (Tween 20 and SDS) as non-nucleic acid denaturants did not increase the luminescence counts significantly compared with the condition of no denaturant (-) (Table 29 and FIG. 20). The chaotropic agents (guanidine thiocyanate and urea) and the electron donating compounds (imidazole and pyrazole) as the nucleic acid denaturants increased the luminescence counts significantly compared with the condition of no denaturant (-) (Table 29 and FIG. 20).
[Table 29]

**Table 29. Measurement of Modified Nucleobase in Target Nucleic Acid Using Guide Probe in Presence of Nucleic Acid Denaturant or Non-Nucleic Acid Denaturant**

| Denaturant | Amount of Target Nucleic Acid | Luminescence Count | S/N |
|---|---|---|---|
| - | 10fmol | 18869 | 19.3 |
| | 1fmol | 4088 | 4.2 |
| | 0mol | 977 | |
| Guanidine Thiocyanate | 10fmol | 154679 | 73.6 |
| | 1fmol | 46165 | 22.0 |
| | 0mol | 2102 | |
| Imidazole | 10fmol | 151900 | 145.1 |
| | 1fmol | 49139 | 46.9 |
| | 0mol | 1047 | |
| Pyrazole | 10fmol | 78880 | 77.0 |
| | 1fmol | 22857 | 22.3 |
| | 0mol | 1024 | |
| Urea | 10fmol | 80014 | 93.4 |
| | 1fmol | 22447 | 26.2 |
| | 0mol | 857 | |
| Tween 20 | 10fmol | 19919 | 24.4 |
| | 1fmol | 3499 | 4.3 |
| | 0mol | 816 | |
| Sodium Lauryl Sulfate (SDS) | 10fmol | 23520 | 25.8 |
| | 1fmol | 3612 | 4.0 |
| | 0mol | 913 | |

| | | | |
|---|---|---|---|
| -: Without Denaturant | | | |

From the foregoing, it has been revealed that the effect of the guide probe can be enhanced by the nucleic acid denaturant but cannot be enhanced by the non-nucleic acid denaturant.

### Reference Example 12: Measurement of Modified Nucleobase Using Guide Probe in the Presence of Both Nucleic Acid Denaturant and Surfactant

Tests were carried out by a method similar to that in Reference Example 11 except that the buffer solution (100 mM of Tris-HCl and 50 mM of EDTA·2Na) not containing the nucleic acid denaturant, 1.5 M of a guanidine thiocyanate (+) buffer solution (100 mM of Tris-HCl and 50 mM of EDTA·2Na), the buffer solution (100 mM of Tris-HCl and 50 mM of EDTA·2Na) containing 1.5 M of guanidine thiocyanate and 1% (v/v) of Tween 20, or the buffer solution (100 mM of Tris-HCl and 50 mM of EDTA·2Na) containing 1.5 M of guanidine thiocyanate and 1% (v/v) of Tween 80 was used when the hybrid including the target nucleic acid, the capture probe, and the guide probe was formed.

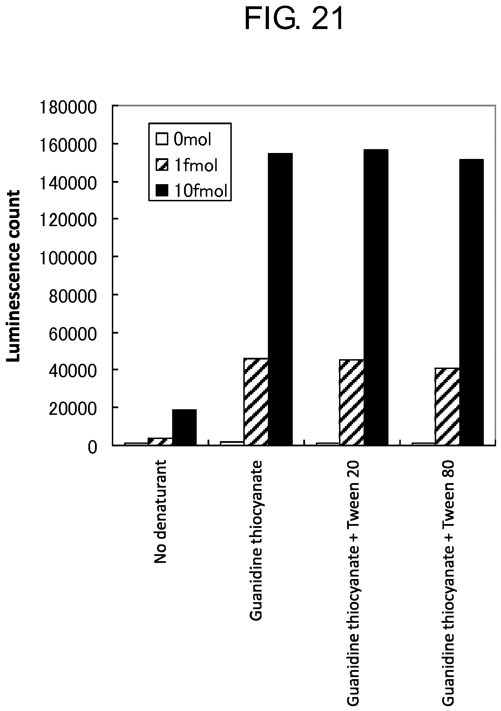

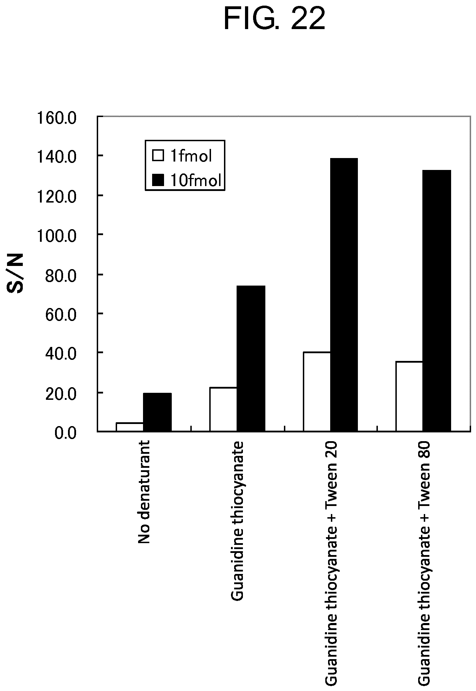

As a result of the measurement, the buffer solution containing the nucleic acid denaturant and the surfactant caused a decrease in the luminescence counts (the background value) and an increase in S/N compared with the buffer solution containing the nucleic acid denaturant alone (Table 30 and FIGs. 21 and 22). In other words, in the method according to the present invention using the guide probe and the nucleic acid denaturant, it is considered that the surfactant has an effect of canceling an increase in the background value caused by the nucleic acid denaturant.
[Table 30]

**Table 30. Measurement of Modified Nucleobase Using Guide Probe in Presence of both Nucleic Acid Denaturant and Surfactant**

| Nucleic acid Denaturant ± Surfactant | Amount of Target Nucleic Acid | Luminescence Count | S/N |
|---|---|---|---|
| - | 10fmol | 18869 | 19.3 |
| | 1fmol | 4088 | 4.2 |
| | 0mol | 977 | |
| Guanidine Thiocyanate | 10fmol | 154679 | 73.6 |
| | 1fmol | 46165 | 22.0 |
| | 0mol | 2102 | |
| Guanidine Thiocyanate+Tween20 | 10fmol | 156474 | 138.7 |
| | 1fmol | 45393 | 40.2 |
| | 0mol | 1128 | |
| Guanidine Thiocyanate+Tween80 | 10fmol | 151366 | 132.4 |
| | 1fmol | 40724 | 35.6 |
| | 0mol | 1143 | |

| | | | |
|---|---|---|---|
| -: Without Denaturant | | | |

From the foregoing, it has been revealed that the guide probe can measure the modified nucleobase in the target nucleic acid with high sensitivity in the presence of both the nucleic acid denaturant and the surfactant.

### (Details of Guide Probes)

For reference, Table 31 lists details of the guide probes used in the experiments.
[Table 31]

**Table 31. Detail of Guide Probe Used in Experiment Industrial Applicability**

| Guide Probe | Hybridized Region in Target Nucleic Acid (SEQ ID NO:16) (Position from the 5'-end) | Number of Bulge Structure (Number of Unpaired Methylated Cytosine) | Distance to Capture Probe in Hybrid |
|---|---|---|---|
| 1 | 1- to 110-Positions | 14 | 3 Nucleotide Residues |
| 2 | 75- to 111-Positions | 4 | 2 Nucleotide Residues |
| 3 (or 5) | 54- to 73-Positions | 0 | 40 Nucleotide Residues |
| 4 (or 6) | 7- to 52-Positions | 8 | 61 Nucleotide Residues |

The method and the kit according to the present invention are useful for measuring a target nucleic acid containing a modified nucleobase.

## Claims

1. A method for measuring a target nucleic acid containing a modified nucleobase, the method comprising:
(1) incubating a nucleic acid sample and a heterologous nucleic acid probe having a property of pairing with a modified nucleobase, in a solution; and
(2) measuring the modified nucleobase using an antibody against a modified nucleobase having a property of heterologous pairing, in the solution obtained at (1).

2. The method according to claim 1, wherein
the nucleic acid sample contains the target nucleic acid containing the modified nucleobase, and
steps (1) and (2) are performed by (1') and (2'), respectively:
(1') reacting the nucleic acid sample containing the target nucleic acid containing the modified nucleobase with the heterologous nucleic acid probe having a property of pairing with the modified nucleobase, in a solution by incubation, to form a heterologous nucleic acid hybrid including said target nucleic acid and said probe; and
(2') measuring the modified nucleobase using said antibody in the solution containing said heterologous nucleic acid hybrid.

3. The method according to claim 1 or 2, further comprising adding said probe to a solution containing said nucleic acid sample to prepare a solution containing both of said nucleic acid sample and said probe.

4. The method according to any one of claims 1 to 3, wherein said nucleic acid sample is a sample containing a target DNA containing a modified nucleobase.

5. The method according to any one of claims 1 to 4, wherein said probe contains an artificial nucleic acid having a main chain structure different from a main chain structure of the target nucleic acid.

6. The method according to claim 5, wherein said probe is a peptide nucleic acid (PNA) probe or a bridged nucleic acid (BNA) probe.

7. The method according to any one of claims 1 to 6, wherein a nucleobase included in the modified nucleobase is cytosine.

8. The method according to any one of claims 1 to 7, wherein the modified nucleobase is methylcytosine.

9. A method for measuring a target nucleic acid containing a modified nucleobase, the method comprising:
(1) incubating a nucleic acid sample, a heterologous guide probe having a property of pairing with a modified nucleobase, and a capture probe in a solution; and
(2) measuring the modified nucleobase using an antibody against a modified nucleobase having a property of heterologous pairing, in the solution obtained at (1).

10. The method according to claim 9, wherein
the nucleic acid sample contains the target nucleic acid containing the modified nucleobase, and
steps (1) and (2) are performed by (1') and (2'), respectively:
(1') reacting the nucleic acid sample, the heterologous guide probe having a property of pairing with a modified nucleobase, and the capture probe in a solution by incubation to form a heterologous nucleic acid hybrid including said target nucleic acid, said guide probe, and the capture probe; and
(2') measuring the modified nucleobase using the antibody in the solution containing said heterologous nucleic acid hybrid.

11. The method according to claim 10, wherein said guide probe is heterologous to said target nucleic acid; and the capture probe is heterologous to both of said target nucleic acid and said guide probe.

12. The method according to any one of claims 1 to 11, wherein the measurement of the target nucleic acid containing the modified nucleobase using said antibody is performed by ELISA.

13. A kit for measuring a target nucleic acid containing a modified nucleobase, the kit comprising:
(I) a heterologous nucleic acid probe having a property of pairing with a modified nucleobase; and
(II) an antibody against a modified nucleobase having a property of heterologous pairing.

14. The kit for measuring according to claim 13, wherein said probe is a heterologous guide probe having a property of pairing with the modified nucleobase, and the kit further comprises (III) a capture probe.


SEQUENCE LISTING

<110>  FUJIREBIO INC.

<120>  Methods and kit for measuring modified nucleobases in target
       nucleic acid

<130>  PRBA-27751

<150>  JP2014-197719
<151>  2014-09-29

<160>  27

<170>  PatentIn version 3.5

<210>  1
<211>  16
<212>  DNA
<213>  Artificial Sequence

<220>
<223>  DNA sequence of a certain target nucleic acid.  The 1st and 16th
       nucleotides (cytosines) are methylated at position 5.

<400>  1
cgtaggagga ggaagc                                                       16


<210>  2
<211>  16
<212>  DNA
<213>  Artificial Sequence

<220>
<223>  Nucleotide sequence of peptide nucleic acid (PNA) complementary
       to the DNA of SEQ ID NO:1

<400>  2
gcttcctcct cctacg                                                       16


<210>  3
<211>  16
<212>  DNA
<213>  Artificial Sequence

<220>
<223>  DNA sequence of a certain target nucleic acid

<400>  3
cgtaggagga ggaagc                                                       16


<210>  4
<211>  23
<212>  DNA
<213>  Artificial Sequence

<220>
<223>  DNA sequence of a certain target nucleic acid.  The 4th and 13th
       nucleotides (cytosines) are methylated at position 5.

<400>  4
ttgcgcggcg tcctgttgac ttc                                              23


<210>  5
<211>  23
<212>  DNA
<213>  Artificial Sequence

<220>
<223>  DNA sequence of DNA complementary to the DNA of SEQ ID NO:4

<400>  5
gaagtcaaca ggacgccgcg caa                                              23


<210>  6
<211>  16
<212>  DNA
<213>  Artificial Sequence

<220>
<223>  Nucleotide sequence of peptide nucleic acid (PNA) used as a
       negative control

<400>  6
ggtctgtgag tggttc                                                      16


<210>  7
<211>  16
<212>  DNA
<213>  Artificial Sequence

<220>
<223>  DNA sequence of DNA used as a negative control

<400>  7
ccagacactc accaag                                                      16


<210>  8
<211>  15
<212>  DNA
<213>  Artificial Sequence

<220>
<223>  DNA sequence of a certain target nucleic acid.  The 6th and 13th
       nucleotides (cytosines) are methylated at position 5.

<400>  8
tagaacgctt tgcgt                                                       15


<210>  9
<211>  15
<212>  DNA
<213>  Artificial Sequence

<220>
<223>  Nucleotide sequence of peptide nucleic acid (PNA) complementary
       to the DNA of SEQ ID NO:8

<400>  9
acgcaaagcg ttcta                                                        15


<210>  10
<211>  18
<212>  DNA
<213>  Artificial Sequence

<220>
<223>  DNA sequence of a certain target nucleic acid.  The 3rd, 9th,
       12th, and 16th nucleotides (cytosines) are methylated at position
       5.

<400>  10
gtcgttttcg gcggccgc                                                     18


<210>  11
<211>  18
<212>  DNA
<213>  Artificial Sequence

<220>
<223>  Nucleotide sequence of peptide nucleic acid (PNA) complementary
       to the DNA of SEQ ID NO:10

<400>  11
gcggccgccg aaaacgac                                                     18


<210>  12
<211>  18
<212>  DNA
<213>  Artificial Sequence

<220>
<223>  DNA sequence of a certain target nucleic acid.  The 4th, 8th,
       14th, and 16th nucleotides (cytosines) are methylated at position
       5.

<400>  12
gcccgcacgt cctcgcgg                                                     18


<210>  13
<211>  18
<212>  DNA
<213>  Artificial Sequence

<220>
<223>  Nucleotide sequence of peptide nucleic acid (PNA) complementary
       to the DNA of SEQ ID NO:12

<400>  13
ccgcgaggac gtgcgggc                                                     18


<210>  14
<211>  21
<212>  DNA
<213>  Artificial Sequence

<220>
<223>  Forward primer for amplifying the target DNA of SEQ ID NO:16

<400>  14
tagaacgctt tgcgtcccga c                                                 21


<210>  15
<211>  21
<212>  DNA
<213>  Artificial Sequence

<220>
<223>  Reverse primer for amplifying the target DNA of SEQ ID NO:16

<400>  15
ctgcaggacc actcgaggct g                                                 21


<210>  16
<211>  138
<212>  DNA
<213>  Artificial Sequence

<220>
<223>  One of target DNAs to be detected by the method of the present
       invention

<400>  16
tagaacgctt tgcgtcccga cgcccgcagg tcctcgcggt gcgcaccgtt tgcgacttgg      60

tgagtgtctg ggtcgcctcg ctcccggaag agtgcggagc tctccctcgg gacggtggca     120

gcctcgagtg gtcctgca                                                   138


<210>  17
<211>  24
<212>  RNA
<213>  Artificial Sequence

<220>
<223>  Nucleotide sequence of a certain capturing probe to be used in
       the present invention.  The nucleotides that constitute this
       capturing probe are 2'-O-methylated.

<400>  17
ugcaggacca cucgaggcug ccac                                              24


<210>  18
<211>  15
<212>  DNA
<213>  Artificial Sequence

<220>
<223>  Nucleotide sequence of peptide nucleic acid (PNA) used as guide
       probe

<400>  18
acgcaaagcg ttcta                                                        15

<210>  19
<211>  96
<212>  DNA
<213>  Artificial Sequence

<220>
<223>  Guide probe 1

<400>  19
cccagggaga gctcccactc ttccggagca ggcacccaga cactcaccaa gtccaaacgt      60

gccacccagg acctgcggct cggaccaaag cttcta                                96


<210>  20
<211>  34
<212>  DNA
<213>  Artificial Sequence

<220>
<223>  Guide probe 2

<400>  20
tcccagggag agctcccact cttccggagc aggc                                  34


<210>  21
<211>  20
<212>  DNA
<213>  Artificial Sequence

<220>
<223>  Guide probe 3

<400>  21
acccagacac tcaccaagtc                                                  20


<210>  22
<211>  38
<212>  DNA
<213>  Artificial Sequence

<220>
<223>  Guide probe 4

<400>  22
caaacgtgcc acccaggacc tgcggctcgg accaaagc                              38


<210>  23
<211>  34
<212>  RNA
<213>  Artificial Sequence

<220>
<223>  Guide probe 5

<400>  23
ucccagggag agcucccacu cuuccggagc aggc                                  34


<210>  24

<211>  38
<212>  RNA
<213>  Artificial Sequence

<220>
<223>  Guide probe 6

<400>  24
caaacgugcc acccaggacc ugcggcucgg accaaagc                              38


<210>  25
<211>  15
<212>  DNA
<213>  Artificial Sequence

<220>
<223>  Nucleotide sequence of DNA comprising a briged nucleic acid at
       position 10 complementary to the DNA of SEQ ID NO:8

<400>  25
acgcaaagcg ttcta                                                       15


<210>  26
<211>  15
<212>  DNA
<213>  Artificial Sequence

<220>
<223>  Nucleotide sequence of DNA complementary to the DNA of SEQ ID
       NO:8

<400>  26
acgcaaagcg ttcta                                                       15


<210>  27
<211>  15
<212>  DNA
<213>  Artificial Sequence

<220>
<223>  DNA sequence of a certain target nucleic acid

<400>  27
tagaacgctt tgcgt                                                       15